# EUROPEAN PATENT APPLICATION

(11) **EP 0 949 271 A1**
(43) Date of publication of application: **13.10.1999**
(21) Application number: 97946126.6
(22) Date of filing: 05.12.1997
(51) Int. Cl.: C07K 14/54, C12N 15/24, C12N 5/10, C12N 5/20, C07K 16/24, G01N 33/577, G01N 33/50, A61K 39/00, A61K 38/19, C12Q 1/68, C12Q 1/04, C12P 21/08, C12P 21/02, C12N 15/06

(54) **NOVEL DNA, NOVEL PROTEIN, AND NOVEL ANTIBODY**

(30) Priority: 05.12.1996 JP 32576296
(71) Applicant: KYOWA HAKKO KOGYO CO., Ltd., Chiyoda-ku, Tokyo 100 (JP)
(72) Inventor: YOSHISUE, Hajime, Machida-shi, Tokyo 194 (JP); SAITO, Akiko, Machida-shi, Tokyo 194 (JP); NAKAGAWA, Satoshi, Machida-shi, Tokyo 194 (JP); KUGA, Tetsuro, Machida-shi, Tokyo 194 (JP); SHINKAI, Akeo, Machida-shi, Tokyo 194 (JP); KOIKE, Masamichi, Machida-shi, Tokyo 194 (JP); NISHI, Tatsunari, Ohta-ku, Tokyo 145 (JP)
(74) Representative: VOSSIUS & PARTNER
(86) International application number: JP9704470
(87) International publication number: WO9824817

(57) **Abstract**

A novel protein capable of activating eosinophile cells; a DNA or oligonucleotides encoding this protein; a recombinant vector containing this DNA; a transformant containing this recombinant vector; a process for producing the above protein by using this transformant; a cell reacting specifically with the above protein; a cell membrane or a receptor binding specifically to the above protein; an agonist or an antagonist to the protein; an antibody binding specifically to the protein; and remedies or diagnostic methods with the use of the same for allergic inflammation, eosinophilic pneumonia, sudden eosinophilia, autoimmune disease, malignant tumor, or vermination.

## Description

### Technical Field

The present invention relates to a protein capable of activating eosinophils; a DNA or an oligonucleotide coding for said protein; a recombinant vector comprising said DNA; a transformant carrying said recombinant vector; a process for producing a protein using said transformant; a cell which is specifically acted on by said protein; a cell membrane or a receptor which specifically binds to said protein; an agonist or an antagonist of said protein; an antibody which specifically binds to said protein; and a therapeutic agent or a diagnostic method for allergic inflammation, eosinophilic pneumonia, hypereosinophilic syndrome, autoimmune diseases, malignant tumors and parasitism utilizing them.

### Background Art

Vascular endothelial cells constitute the inner surface of blood vessels and thus are in direct contact with blood cells. When inflammation occurs, leukocytes adhere to and accumulate on the vascular walls and then infiltrate into tissues. Vascular endothelial cells play an important role in inducing this adhesion of leukocytes by expressing adhesion molecules and chemokines. The expression of adhesion molecules and chemokines by vascular endothelial cells is reported to be promoted or inhibited by cytokines secreted by leukocytes. For example, it has been reported that tumor necrosis factor α (hereinafter referred to as TNF-α) increases the expression of E-selectin, which is an adhesion molecule, and interleukin-8 (IL-8), which is a chemokine [Science, 243, 1160 (1989); EMBO Journal, 13, 843 (1994)].

Many kinds of cytokines are concerned in the mechanism of inflammation, and interleukin-4 (IL-4) secreted by activated T cells and mast cells is considered to be one of such cytokines. IL-4 was initially reported as a B cell growth differentiation factor, and afterwards reports have been made of its various actions on a variety of cells including blood cells. For example, observations of IL-4 knock out mouse have revealed that IL-4 has the following activities *in vivo*: IL-4 differentiates T cells to Type 2 helper T cells, and through secretion of cytokines such as IL-4, IL-5 and IL-6 by Type 2 helper T cells, causes the immune system to tend to be on the humoral immune response side; and IL-4 participates in the class switching associated with antibody production by B cells to promote production of IgE and IgG1 [Blood, 77, 1859 (1991); Science, 254, 707 (1991); Nature, 362, 245 (1993)].

Further, it is known that eosinophils infiltration plays a major role in the development of symptoms in allergic inflammatory diseases such as asthma, and IL-4 is reported to be deeply concerned in this eosinophils infiltration. For example, there have been a report that transplantation of cancer cells capable of IL-4 expression into a mouse induced accumulation of eosinophils and macrophage on the transplanted part to cause death of the cancer cells [Cell, 57, 503 (1989)]; a report that in a transgenic mouse in which IL-4 was expressed, eosinophils and mast cells accumulated on the eyelids to induce allergic inflammation [Cell, 62, 457 (1990)]; a report that in a transgenic mouse in which IL-4 was expressed lung-specifically, eosinophils and mast cells accumulated in the lung to induce asthma-like symptoms such as thickening of epithelial cells [Proc. Natl. Acad. Sci., 93, 7821 (1996)]; a report that vascular inflammation accompanying eosinophils infiltration was observed in a monkey that received continuous subcutaneous administration of IL-4 [Toxicologic Pathology, 19, 251 (1991)]; and a report that the number of eosinophils infiltrating in allergic reaction caused by antigenic sensitization is decreased in IL-4 gene knock out mouse [Nature, 362, 245 (1993)]. These results reported suggest that molecules relating to allergic inflammation or eosinophils infiltration are present in the molecules whose expression is induced by IL-4 in vascular endothelial cells. Adhesion molecules which participate in the adhesion of eosinophils to endothelial cells and chemokines which participate in the activation or wandering of eosinophils are considered to play an important role in eosinophils infiltration into allergic inflammation regions. It has been reported that the expression of VCAM-1 (vascular cell adhesion molecule-1), L-selectin ligand and p-selectin, which are adhesion molecules, is increased by stimulating vascular endothelial cells with IL-4 [J. Immunol., 148, 1086 (1992); J. Cell Biol., 125, 1417 (1994); J. Exp. Med., 184, 81 (1996)]. It has also been reported that stimulation of vascular endothelial cells with IL-4 caused induction of MCP-1 (monocyte chemoattractant protein-1), which is a chemokine, at the mRNA level [American Journal of Pathology, 138, 1315 (1991)].

Chemokines are molecules having the activity to cause leukocytes to wander in the direction of higher chemokine concentration via receptors, and thus play an important role in the infiltration of leukocytes. It is known that the binding of chemokines to receptors brings about the increase of intracellular calcium concentration. Further, chemokines are reported to have the activity to activate leukocytes as well as the activity to induce cell wandering; for example, reports have been made of the activity to induce activation of integrin, which is an adhesion molecule, and the activity to promote degranulation and proliferation of leukocytes [Nature, 361, 79 (1993); Journal of Leukocyte Biology, 59, 81 (1996)]. From the foregoing, chemokines are considered to be molecules which are deeply concerned not only in the above-mentioned inflammation but also in diseases whose progress is supposed to be associated with the activation and wandering of leukocytes, e.g. arteriosclerosis in which accumulation of foam macrophage on vascular smooth muscles through vascular endothelial cells is observed, and autoimmune diseases in which activated lymphocytes act as a self-injuring factor. Some chemokines are reported to have growth inhibitory effect on hematopoietic stem cells [Nature, 344, 442 (1990)]. Further, a report has been made of the inhibitory effect of three kinds of chemokines, i.e., RANTES, MIP-1α (macrophage inflammatory protein-1α) and MIP-1β on HIV (human immunodeficiency virus) infection [Science, 270, 1811 (1995)]. On the basis of this report, the relationship between chemokines and HIV infection have been studied and it has been revealed that chemokine receptor CCR5 acts as co-receptor, together with CD4 on cells, against infection with monocyte-tropic (M-tropic) HIV [Nature, 381, 661 (1996); ibid., 381, 667 (1996); Science, 272, 1955 (1996)]. It has also been reported that another chemokine receptor, CXCR4, acts as co-receptor against T cell-tropic (T-tropic) HIV [Science, 272, 872 (1996); Nature, 382, 829 (1996); Nature, 382, 833 (1996)]. So far there have been reported many kinds of chemokines, which are characterized in that four Cys residues are conserved at the same sites in the amino acid sequence. This characteristic suggests that the mode of disulfide bond of Cys residues plays an important role in formation of higher-order structure and retention of chemokine activity. Known chemokines may be classed into two groups based on the amino acid sequence, i.e. CC chemokines having CysCys sequence in the molecule and CXC chemokines having CysXaaCys sequence (Xaa represents an arbitrary amino acid) in the molecule. CC chemokines mostly act on monocytes among leukocytes, whereas CXC chemokines mostly act on neutrophils. However, the kind of target cells and the intensity of action differ with different chemokines, and individual chemokines are different also in regulation of the gene expression. Therefore, it is considered that the secretion of chemokines varies according to the kind of cells and tissues or the stimulation given to them.

Chemokines reported to act on eosinophils include eotaxin, RANTES, MCP-3, MCP-4 and eotaxin-2, among which eotaxin is particularly noted as it acts specifically on eosinophils and its expression is increased in an animal allergy model by antigenic sensitization. However, analysis of the data on eotaxin gene knock out mouse suggests that eotaxin is concerned in the constancy of peripheral eosinophil count in the normal state, but is not concerned in the abnormal eosinophils infiltration as in allergic inflammation except in the initial stage, and chemokines other than eotaxin are also concerned in such eosinophils infiltration in the initial stage [J. Exp. Med., 185, 785 (1997)].

It has been reported that blood eosinophil count is increased by parasitization. On the other hand, eosinophils are known to release various mediators such as proteins in granules, e.g. major basic protein, eosinophil cationic protein and eosinophil peroxidase, and active oxygen by activation. These mediators have cell-killing activity and parasiticidal activity and are supposed to be concerned in the defense against tissue injury by eosinophils and against parasites [Eosinophils: Biological and Clinical Aspects, edited by H. Makino and T. Fukuda, CRC Press (1993)]. Therefore, if it is possible to cause active eosinophils to accumulate on malignant tumor or parasitized regions by the action of chemokines, such diseases will be capable of being effectively treated.

### Disclosure of the Invention

The present invention relates to the following:
(1) a protein having the amino acid sequence represented by SEQ ID NO: 13, or a protein having an ability to activate eosinophils and having an amino acid sequence wherein one or more amino acid residues are deleted, substituted or added in the amino acid sequence represented by SEQ ID NO: 13 (hereinafter referred to as the protein of the present invention);
(2) a DNA coding for the protein of (1), or a DNA which hybridizes to said DNA under stringent conditions and which codes for a protein having an ability to activate eosinophils (hereinafter referred to as the DNA of the present invention);
(3) a recombinant vector comprising the DNA of (2) (hereinafter referred to as the recombinant vector of the present invention);
(4) a transformant which is obtained by introducing the recombinant vector of (3) into a host cell (hereinafter referred to as the transformant of the present invention);
(5) a process for producing a protein which comprises culturing the transformant of (4) in a medium, allowing the above-mentioned protein to accumulate in the culture, and recovering said protein from the culture (hereinafter referred to as the process for producing a protein of the present invention);
(6) a pharmaceutical composition comprising the protein of (1);
(7) a therapeutic agent for malignant tumors or parasitism comprising the protein of (1);
(8) a method of treating malignant tumors or parasitism which comprises administering an effective amount of the protein of (1);
(9) a method for detecting a cell which is specifically acted on by the protein of (1) or a cell membrane or a receptor which specifically binds to the protein of (1), which comprises bringing said protein into contact with a test sample;
(10) a method for obtaining a cell which is specifically acted on by the protein of (1) or a cell membrane or a receptor which specifically binds to the protein of (1), which comprises bringing said protein into contact with a test sample;
(11) a cell, a cell membrane or a receptor which is obtainable by the method of (10);
(12) a method for obtaining an agonist or an antagonist of the protein of (1) which comprises comparing (a) the result obtained by bringing the protein of (1) into contact with the cell, the cell membrane or the receptor of (11) with (b) the result obtained by bringing the protein of (1) into contact with the cell, the cell membrane or the receptor of (11) and a test compound;
(13) an agonist or an antagonist which is obtainable by the method of (12) (hereinafter referred to as the agonist or the antagonist of the present invention);
(14) an antibody which specifically reacts with the protein of (1) (hereinafter referred to as the antibody of the present invention);
(15) hybridoma KM1885;
(16) a method of immunologically detecting or determining the protein of (1) using the antibody of (14);
(17) a diagnostic method for allergic inflammation, eosinophilic pneumonia, hypereosinophilic syndrome or autoimmune diseases using the antibody of (14);
(18) a diagnostic agent for allergic inflammation, eosinophilic pneumonia, hypereosinophilic syndrome or autoimmune diseases comprising the antibody of (14);
(19) a pharmaceutical composition comprising the antibody of (14);
(20) a therapeutic agent for allergic inflammation, eosinophilic pneumonia, hypereosinophilic syndrome or autoimmune diseases comprising the antibody of (14);
(21) a method of treating allergic inflammation, eosinophilic pneumonia, hypereosinophilic syndrome or autoimmune diseases which comprises administering an effective amount of the antibody of (14);
(22) a sense DNA of (2);
(23) an antisense DNA of (2);
(24) an oligonucleotide comprising a part of the nucleotide sequence of the DNA of (22);
(25) an oligonucleotide comprising a part of the nucleotide sequence of the DNA of (23);
(26) a method of detecting an mRNA coding for the protein of (1) using the DNA of (22) or (23) or the oligonucleotide of (24) or (25);
(27) a diagnostic method for allergic inflammation, eosinophilic pneumonia, hypereosinophilic syndrome, autoimmune diseases, malignant tumors or parasitism using the DNA of (22) or (23) or the oligonucleotide of (24) or (25);
(28) a diagnostic agent for allergic inflammation, eosinophilic pneumonia, hypereosinophilic syndrome, autoimmune diseases, malignant tumors or parasitism comprising the DNA of (22) or (23) or the oligonucleotide of (24) or (25);
(29) a method for repressing the expression of the protein of (1) using the DNA of (23) or the oligonucleotide of (25);
(30) a pharmaceutical composition comprising the DNA of (23) or the oligonucleotide of (25);
(31) a therapeutic agent for allergic inflammation, eosinophilic pneumonia, hypereosinophilic syndrome or autoimmune diseases comprising the DNA of (23) or the oligonucleotide of (25);
(32) a method of treating allergic inflammation, eosinophilic pneumonia, hypereosinophilic syndrome or autoimmune diseases which comprises administering an effective amount of the DNA of (23) or the oligonucleotide of (25);
(33) a vector for the gene therapy for allergic inflammation, eosinophilic pneumonia, hypereosinophilic syndrome, autoimmune diseases, malignant tumors or parasitism comprising the DNA of (22) or (23) or the oligonucleotide of (24) or (25) (hereinafter referred to as the vector for the gene therapy of the present invention);
(34) a method of treating allergic inflammation, eosinophilic pneumonia, hypereosinophilic syndrome, autoimmune diseases, malignant tumors or parasitism using the vector for the gene therapy of (33); and
(35) a method for obtaining a DNA coding for a chemokine protein from human vascular endothelial cells stimulated with IL-4 by using the differential display technique.

The present invention is described in detail below.

The expression "having an ability to activate eosinophils" as used herein refers to the capability of acting on eosinophils to promote or enhance the intracellular calcium concentration, intracellular information transmission system or cellular response system.

The proteins of the present invention include the protein having the amino acid sequence represented by SEQ ID NO: 13, and any proteins having the ability to activate eosinophils and having amino acid sequences wherein one or more amino acid residues are deleted, substituted or added in the amino acid sequence represented by SEQ ID NO: 13.

The deletion, substitution or addition of amino acid residues can be carried out by introducing site-specific mutation into DNA coding for the protein having the amino acid sequence represented by SEQ ID NO: 13 using the site-specific mutagenesis techniques described in Nucleic Acids Research, 10, 6487 (1982), Proc. Natl. Acad. Sci., USA, 79, 6409 (1982), Gene, 34, 315 (1985), Nucleic Acids Research, 13, 4431 (1985), Proc. Natl. Acad. Sci., USA, 82, 488 (1985), etc. The number of amino acid residues which are deleted, substituted or added is not specifically limited, but is preferably within the range of one to several decades, more preferably one to several, according to the procedure of site-specific mutagenesis. In order that the protein of the present invention has the ability to activate eosinophils, it is preferred that the protein has an amino acid sequence showing at least 60% homology, normally 80% or more homology, particularly 95% or more homology to the amino acid sequence represented by SEQ ID NO: 13. Further, in order that the protein of the present invention is a chemokine protein, it is preferred that the protein has an amino acid sequence wherein the four Cys residues in the sequence represented by SEQ ID NO: 13 are conserved at the same sites after the substitution, addition or deletion of amino acid residues.

The DNAs of the present invention include DNAs coding for the proteins of the present invention, for example, DNA having the nucleotide sequence represented by SEQ ID NO: 14 which codes for the protein having the amino acid sequence represented by SEQ ID NO: 13. Generally, there exist plural genetic codes corresponding to a single amino acid, and DNAs having nucleotide sequences other than that of SEQ ID NO: 14 and coding for the protein having the amino acid sequence represented by SEQ ID NO: 13 are also included in the DNAs of the present invention.

The DNA which hybridizes to the DNA coding for the protein of the present invention under stringent conditions refers to DNA which is obtained by colony hybridization, plaque hybridization or Southern blot hybridization using the DNA coding for the protein of the present invention such as DNA having the nucleotide sequence represented by SEQ ID NO: 14. For example, such DNA can be obtained in the following manner. Colony- or plaque-derived DNA immobilized on a filter is subjected to hybridization at 65°C overnight in a hybridization solution {6 x SSC [0.9 M NaCl, 90 mM sodium citrate; n x SSC refers to n-fold concentration of 1 x SSC (150 mM NaCl, 15 mM sodium citrate)], 5 x Denhalt solution (0.1% bovine serum albumin, 0.1% Ficoll, 0.1% polyvinyl pyrrolidone), 0.5% sodium dodecyl sulfate (SDS), 20 µg/ml denatured DNA obtained by subjecting salmon sperm DNA to ultrasonication, heating in a boiling water bath for 5 minutes, and then rapid cooling in ice} containing ³²P-labeled DNA having the nucleotide sequence represented by SEQ ID NO: 14 as a probe. After washing by shaking in 2 x SSC at 65°C for 15 minutes is repeated twice, the resulting mixture is subjected to washing by shaking in 2 x SSC containing 0.1% SDS at 65°C for 30 minutes, and then washing by shaking in 0.1 x SSC containing 0.1% SDS at 65°C for 10 minutes, whereby DNA which hybridized to said probe can be obtained.

The DNA of the present invention can be obtained by the following method.

A cDNA fragment of the protein of the present invention can be obtained by analyzing a gene whose mRNA expression in human vascular endothelial cells, e.g. human umbilical cord vein vascular endothelial cells, stimulated with IL-4 differs in degree from that in the same cells not stimulated with IL-4, by using the differential display technique. By screening of the vascular endothelial cell cDNA library using the obtained cDNA fragment as a probe, cloning of the DNA of the present invention can be carried out.

The method for preparing the DNA of the present invention is described below.

It has been clarified that eosinophils play an important role in allergic inflammatory diseases. On the basis of the finding that IL-4 is deeply concerned in the eosinophils infiltration, DNA of a gene relating to the eosinophils infiltration is prepared by analyzing a gene whose mRNA expression level in vascular endothelial cells changes by IL-4 stimulation of the cells by using the differential display technique [Science, 257, 967 (1992); FEBS Letters, 351, 231 (1994)].

First, total RNA is prepared from vascular endothelial cells which were stimulated with IL-4 and vascular endothelial cells which were not stimulated with IL-4 according to the guanidine thiocyanate-cesium trifluoroacetate method [Methods in Enzymology, 154, 3 (1987)], the acidic guanidine thiocyanate-phenol-chloroform (AGPC) method [Analytical Biochemistry, 162, 156 (1987)], etc.

The obtained RNA is subjected to reaction with reverse transcriptase using an anchor primer according to the methods described in Molecular Cloning, A Laboratory Manual, Second Edition, Cold Spring Harbor Laboratory Press (1989), Current Protocols in Molecular Biology, Supplement 1-38, John Wiley & Sons (1987-1997), DNA Cloning 1: Core Techniques, A Practical Approach, Second Edition, Oxford University Press (1995), etc. to synthesize cDNA from mRNA in the RNA.

Then, each cDNA is subjected to polymerase chain reaction (hereinafter referred to as PCR) using the anchor primer and another primer to amplify cDNA fragments. The anchor primer is a primer prepared by adding an oligonucleotide consisting of adenine, guanine and cytosine (excluding thymidine) to the 3' end of an oligo dT sequence which hybridizes to the 3' end poly A sequence of mRNA. An example of suitable anchor primer is the oligonucleotide having the nucleotide sequence of SEQ ID NO: 4.

As the other primer, oligonucleotides capable of amplifying various kinds of cDNA and giving many cDNA fragments by one reaction can be used. Examples of such primers include OPA-1∼20, OPB-1∼20, OPC-1∼20 and OPD-1∼20 (Operon Technologies). It is preferred that this primer consists of 10-20 bases.

After the cDNA fragments are amplified by PCR, the reaction mixture is subjected to polyacrylamide gel electrophoresis and the cDNA fragments in the gel are fluorescence-stained with a DNA-specific fluorescent staining agent such as Cyber Green I. The fluorescence intensity of each fragment can be determined by using FluorImager and can be visualized as light and shade band patterns. By the use of an anchor primer whose 5' end is fluorescence-labeled in PCR, the fluorescence intensity of the amplified fragments can be determined immediately after the electrophoresis without fluorescence staining. Fluorescence-labeling of the 5' end of a primer can be carried out by using fluorescein isothiocyanate according to an ordinary method. After the cDNA fragments obtained by amplification of RNA prepared from IL-4-stimulated vascular endothelial cells and non-IL-4-stimulated vascular endothelial cells are subjected to electrophoresis, the fluorescence intensity of each band is determined, and the gel portion corresponding to the band showing the difference in fluorescence intensity due to IL-4 stimulation is cut out.

By PCR using a part of the gel cut out as a template, cDNA fragments in the gel are amplified. The amplified fragments are treated with Pfu DNA polymerase, etc. to make the ends blunt ends if necessary, and then introduced into a vector for cloning. Suitable vectors for the introduction of the amplified cDNA fragments include pT7Blue T-Vector (Novagen), pDIRECT [Clontech, Nucleic Acids Research, 18, 6069 (1990)], pCR-Script Amp (Stratagene), pCR2.1 (Invitrogen), pCR-TRAP (GenHunter) and pTA (Nippon Gene).

The nucleotide sequences of the cloned cDNA fragments are determined by the dideoxy method of Sanger, et al. [Proc. Natl. Acad. Sci. USA, 74, 5463 (1977)] or by using DNA sequencers produced by Perkin Elmer, Pharmacia, etc. The novelty of the thus determined nucleotide sequences can be confirmed by the absence of a nucleotide sequence showing obvious homology which is considered to indicate the agreement with any of the nucleotide sequences in the data bases of GenBank, EMBL, DDBJ, etc. according to the search of these data bases by using the homology search program of BLAST, etc.

An example of the DNA having a novel nucleotide sequence obtained as above is the DNA having the nucleotide sequence represented by SEQ ID NO: 3. This DNA was obtained from a band showing an increased expression in IL-4-stimulated vascular endothelial cells compared with non-IL-4-stimulated vascular endothelial cells, and as a result of the analysis described below, was found to be the DNA obtained by amplification of a part of the cDNA coding for HVC002 protein, which is the protein of the present invention whose expression is increased in IL-4-stimulated vascular endothelial cells.

The DNA of the present invention can be obtained by screening of cDNA library or RACE (Rapid Amplification of cDNA Ends) utilizing the amplified DNA fragment having the nucleotide sequence of SEQ ID NO: 3 obtained above.

From the total RNA obtained from IL-4-stimulated vascular endothelial cells by the above method, mRNA is prepared as poly(A)⁺RNA according to the method using oligo (dT) cellulose [Molecular Cloning, A Laboratory Manual, Second Edition, Cold Spring Harbor Laboratory Press (1989)], etc. Alternatively, mRNA may be prepared directly from IL-4-stimulated vascular endothelial cells by using Fast Track mRNA Isolation Kit (Invitrogen), Quick Prep mRNA Purification Kit (Pharmacia), etc. The obtained mRNA is converted to cDNA, inserted into a suitable vector, and then introduced into a host cell to prepare cDNA library. The cDNA library can be prepared according to the methods described in Molecular Cloning, A Laboratory Manual, Second Edition (1989), Current Protocols in Molecular Biology, Supplement 1-38 (1987-1997), DNA Cloning 1: Core Techniques, A Practical Approach, Second Edition (1995), etc. or by using commercially available kits such as SuperScript Plasmid System for cDNA Synthesis and Plasmid Cloning (Life Technologies) and ZAP-cDNA Synthesis Kit (Stratagene). As the cloning vector for preparing the cDNA library, any of phage vectors and plasmid vectors can be used so long as it is capable of autonomous replication in Escherichia coli K12. Examples of suitable vectors are ZAP Express (Stratagene), pBluescript II SK(+) [Nucleic Acids Research, 17, 9494 (1989)], Lambda ZAP II (Stratagene), λ gt10, λ gt11 [DNA Cloning, A Practical Approach, 1, 49 (1985)], λ TriplEx (Clontech), λ ExCell (Pharmacia), pT7T318U (Pharmacia) and pcD2 (Mol. Cell. Biol., 3, 280 (1983)].

As the host microorganism, any microorganism belonging to the species Escherichia coli can be used. Examples of suitable host microorganisms are Escherichia coli XL1-Blue MRF' (Stratagene), Escherichia coli C600 [Genetics, 39, 440 (1954)], Escherichia coli Y1088 [Science, 222, 778 (1983)], Escherichia coli Y1090 [Science, 222, 778 (1983)], Escherichia coli NM522 [J. Mol. Biol., 166, 1 (1983)], Escherichia coli K802 [J. Mol. Biol., 16, 118 (1966)] and Escherichia coli JM105 [Gene, 38, 275 (1985)]. The cDNA library obtained as above is subjected to colony hybridization or plaque hybridization [Molecular Cloning, A Laboratory Manual, Second Edition (1989), Current Protocols in Molecular Biology, Supplement 1-38 (1987-1997), DNA Cloning 1: Core Techniques, A Practical Approach, Second Edition (1995)] to obtain cDNA clone coding for the protein of the present invention. As the probe, a DNA fragment having the nucleotide sequence of SEQ ID NO: 3 which is labeled with ³²P or the like can be used.

The nucleotide sequence of the obtained cDNA can be determined by the above-mentioned methods.

An example of the DNA coding for the protein of the present invention obtained by the above method is the DNA having the nucleotide sequence represented by SEQ ID NO: 14.

By the use of RACE technique, cDNA which is upstream (5' end side) from the amplified fragment can be obtained [Proc. Natl. Acad. Sci. USA, 85, 8998 (1988)]. That is, cDNA is synthesized from IL-4-stimulated vascular endothelial cells in the same manner as in the screening of cDNA library, and after addition of an adapter to both ends of this cDNA, PCR is carried out using primers prepared based on the nucleotide sequence of the adapter and the nucleotide sequence of the amplified fragment. The thus amplified fragment is cloned into a vector in the same manner as described above, whereby the cDNA upstream from the amplified fragment obtained by differential display can be obtained. The nucleotide sequence of the obtained cDNA can be determined in the same manner as in the screening of cDNA library. On the basis of this nucleotide sequence, the obtained cDNA and the amplified fragment obtained by differential display can be ligated to give cDNA which is considered to have the entire sequence.

After the nucleotide sequence of the DNA of the present invention is clarified in the above manner, DNA coding for the protein of the present invention can be obtained by PCR [PCR, A Practical Approach (1991)] using a primer prepared based on the nucleotide sequence of the above cDNA and using, as a template, cDNA or cDNA library prepared from IL-4-stimulated vascular endothelial cells in the same manner as above.

The DNA of the present invention can also be prepared by chemical synthesis based on the determined nucleotide sequence using a DNA synthesizer such as DNA synthesizer model 392 (Perkin Elmer) utilizing the phosphoamidite method.

By using a DNA synthesizer, an antisense oligonucleotide having a part of the nucleotide sequence of the DNA of the present invention can be chemically synthesized. Derivatives of such nucleotide, e.g. methylated nucleotide and phosphorothioate nucleotideanalog, can also be used in the present invention.

The DNA of the present invention obtained by the above method can be expressed in a host cell according to the methods described in Molecular Cloning, A Laboratory Manual, 2nd Ed. (1989), Current Protocols in Molecular Biology, Supplement 1-38 (1987-1997), etc.

That is, the DNA of the present invention is inserted into an appropriate expression vector at an insertion site located downstream of the promoter therein to construct the recombinant vector of the present invention, and the obtained recombinant vector is introduced into a host cell, whereby the transformant of the present invention can be obtained.

As the host cell, any bacterial cells, yeast cells, animal cells, insect cells, etc. which are capable of expressing the desired gene can be used. As the expression vector, vectors capable of autonomous replication or integration into chromosome in the above host cells and comprising a promoter at a site appropriate for the transcription of the DNA of the present invention are used.

When a procaryotic cell such as a bacterial cell is used as the host cell, it is preferred to use the recombinant vector of the present invention which is capable of autonomous replication in a procaryotic cell and which comprises a promoter, a ribosome binding sequence, the DNA of the present invention, and a transcription termination sequence. The vector may further comprise a gene regulating the promoter.

Examples of suitable expression vectors are pKK233-2 (Pharmacia), pSE280 (Invitrogen), pGEMEX-1 (Promega), pQE-8 (QIAGEN), pKYP10 (Japanese Published Unexamined Patent Application No. 110600/83), pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (Stratagene), pGEX (Pharmacia) and pET-3 (Novagen). Preferred expression vectors are those in which the distance between the Shine-Dalgarno sequence (ribosome binding sequence) and the initiation codon is adjusted to an appropriate length (e.g. 6-18 bases).

As the promoter, any promoters capable of expression in host cells such as E. coli can be used. For example, promoters derived from E. coli or phage, such as trp promoter (Pₜᵣₚ), lac promoter, P_{L} promoter, P_{R} promoter and T7 promoter, may be used. Artificially modified promoters such as a promoter in which two Pₜᵣₚs are combined in tandem (Pₜᵣₚ x 2), tac promoter, lacT7 promoter and let I promoter can also be used.

In the recombinant vector of the present invention, the transcription termination sequence is not essential for the expression of the DNA of the present invention, but it is preferred that the transcription termination sequence be located immediately downstream of the structural gene.

Examples of suitable host cells are cells of microorganisms belonging to the genus Escherichia, Bacillus, Corynebacterium, Brevibacterium, Pseudomonas or Serratia, specifically, Escherichia coli XL1-Blue MRF', Escherichia coli DH1, Escherichia coli JM109, Escherichia coli HB101, Bacillus subtilis, Bacillus amyloliquefaciens, Brevibacterium immariophilum ATCC 14068, Brevibacterium saccharolyticum ATCC 14066, Corynebacterium glutamicum ATCC 13032, and Corynebacterium acetoacidophilum ATCC 13870.

Introduction of the recombinant vector can be carried out by any of the methods for introducing DNA into the above host cells, for example, the calcium chloride method [Proc. Natl. Acad. Sci. USA, 69, 2110 (1972)], the protoplast method (Japanese Published Unexamined Patent Application No. 2483942/88) and the electroporation method.

When a yeast cell is used as the host cell, YEp13 (ATCC 37115), YEp24 (ATCC 37051), YCp50 (ATCC 37419), etc. can be used as the expression vector.

As the promoter, any promoters capable of expression in yeast cells can be used. Examples of suitable promoters are promoters of genes in the glycolytic pathway such as hexose kinase, gal 1 promoter, gal 10 promoter, heat shock polypeptide promoter, MFα1 promoter, and CUP 1 promoter.

Examples of suitable host cells are cells of Saccharomyces cerevisiae, Schizosaccharomyces pombe, Kluyveromyces lactis, Trichosporon pullulans, and Schwanniomyces alluvius.

Introduction of the recombinant vector can be carried out by any of the methods for introducing DNA into yeast cells, for example, the electroporation method [Methods. Enzymol., 194, 182 (1990)], the spheroplast method [Proc. Natl. Acad. Sci. USA, 81, 4889 (1984)], and the lithium acetate method [Journal of Bacteriology, 153, 163 (1983)].

When an animal cell is used as the host cell, pAGE107 [Japanese Published Unexamined Patent Application No. 22979/91, Cytotechnology, 3, 133 (1990)], pAS3-3 (Japanese Published Unexamined Patent Application No. 227075/90), pCDM8 [Nature, 329, 840 (1987)], pcDNAI/Amp (Invitrogen), pREP4 (Invitrogen), pAGE103 [Journal of Biochemistry, 101, 1307 (1987)], etc. can be used as the expression vector.

As the promoter, any promoters capable of expression in animal cells can be used. Examples of suitable promoters are the promoter of IE (immediate early) gene of cytomegalovirus (CMV), SV40 early promoter and metallothionein promoter. The enhancer of IE gene of human CMV may be used in combination with the promoter.

Examples of suitable host cells are human-derived Namalwa cell, monkey-derived COS cell, Chinese hamster-derived CHO cell and HBT5637 (Japanese Published Unexamined Patent Application No. 299/88).

Introduction of the recombinant vector can be carried out by any of the methods for introducing DNA into animal cells, for example, the electroporation method [Cytotechnology, 3, 133 (1990)], the calcium phosphate method (Japanese Published Unexamined Patent Application No. 227075/90), and the lipofection method [Proc. Natl. Acad. Sci. USA, 84, 7413 (1987)].

When an insect cell is used as the host cell, a protein can be expressed by using the methods described in Baculovirus Expression Vectors, A Laboratory Manual, W. H. Freeman and Company, New York (1992), Current Protocols in Molecular Biology, Supplement 1-38 (1987-1997), Bio/Technology, 6, 47 (1988), etc.

That is, the recombinant vector of the present invention (hereinafter referred to also as the recombinant gene transfer vector of the present invention) and a baculovirus are introduced into an insect cell to obtain a recombinant virus in the culture supernatant of the insect cell, and then an insect cell is infected with the recombinant virus, whereby the protein can be expressed.

Examples of the gene transfer vectors suitable for use in this method are pVL1392, pVL1393 and pBlueBacIII (products of Invitrogen).

An example of the baculovirus is Autographa californica nuclear polyhedrosis virus, which is a virus infecting insects belonging to the family Barathra.

Examples of the insect cells are Sf9 and Sf21 [Baculovirus Expression Vectors, A Laboratory Manual, New York (1992)], which are ovary cells of Spodoptera frugiperda, and High 5 (Invitrogen), which is an ovary cell of Trichoplusia ni.

Introduction of the recombinant gene transfer vector of the present invention and the baculovirus into an insect cell for the preparation of the recombinant virus can be carried out by the calcium phosphate method (Japanese Published Unexamined Patent Application No. 227075/90), the lipofection method [Proc. Natl. Acad. Sci. USA, 84, 7413 (1987)], etc.

Expression of the gene can be carried out not only by direct expression but also by secretory production, fused protein expression, etc. according to the methods described in Molecular Cloning, A Laboratory Manual, Second Edition (1989), etc.

When the gene is expressed in a yeast cell, an animal cell or an insect cell, a sugar- or sugar chain-attached protein can be obtained.

The protein of the present invention can be produced by culturing the transformant of the present invention in a medium, allowing the protein of the present invention to accumulate in the culture, and recovering the protein from the culture. Culturing of the transformant of the present invention can be carried out by conventional methods for culturing the host cell of the transformant.

For the culturing of the transformant prepared by using a procaryotic cell such as E. coli cell or a eucaryotic cell such as a yeast cell as the host cell, any of natural media and synthetic media can be used insofar as it is a medium suitable for efficient culturing of the transformant which contains carbon sources, nitrogen sources, inorganic salts, etc. which can be assimilated by the host used.

As the carbon sources, any carbon sources which can be assimilated by the host can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of inorganic or organic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented cells and digested products thereof.

Examples of the inorganic substances include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged aeration stirring culture, at 15-40°C for 16-96 hours. The pH is maintained at 3.0-9.0 during the culturing. The pH adjustment is carried out by using an organic or inorganic acid, an alkali solution, urea, calcium carbonate, ammonia, etc. If necessary, antibiotics such as ampicillin and tetracycline may be added to the medium.

When a microorganism transformed with an expression vector comprising an inducible promoter is cultured, an inducer may be added to the medium, if necessary. For example, in the case of a microorganism transformed with an expression vector comprising lac promoter, isopropyl-β-D-thiogalactopyranoside or the like may be added to the medium; and in the case of a microorganism transformed with an expression vector comprising trp promoter, indoleacrylic acid or the like may be added.

For the culturing of the transformant prepared by using an animal cell as the host cell, RPMI1640 medium, Eagle's MEM medium, media prepared by adding fetal calf serum to these media, etc. can be used as the medium.

Culturing is usually carried out in the presence of 5% CO₂ at 35-37°C for 3-7 days.

If necessary, antibiotics such as kanamycin and penicillin may be added to the medium.

For the culturing of the transformant prepared by using an insect cell as the host cell, TNM-FH medium (Pharmingen), Sf-900 II SFM medium (Life Technologies), ExCell400 and ExCell405 (JRH Biosciences), etc. can be used as the medium.

Culturing is usually carried out at 25-30°C for 1-4 days. If necessary, antibiotics such as gentamicin may be added to the medium.

The protein expressed in the above-described manner can be isolated and purified from the culture of the transformant by conventional methods for isolating and purifying proteins.

For example, when the protein of the present invention is expressed in cells in a dissolved state, the isolation and purification can be carried out in the following manner. After the completion of culturing, the cells are separated from the culture by centrifugation and suspended in an aqueous buffer, followed by disruption using an ultrasonic disruptor, a French press, a Manton Gaulin homogenizer, a Dyno Mill, etc. to obtain a cell-free extract. The cell-free extract is centrifuged, and from the obtained supernatant, a purified protein preparation can be produced by using ordinary means for isolation and purification of proteins, for example, extraction with a solvent, salting-out with ammonium sulfate, etc., desalting, precipitation with an organic solvent, anion exchange chromatography using resins such as diethylaminoethyl (DEAE)-Sepharose and DIAION HPA-75 (Mitsubishi Kasei Corporation), cation exchange chromatography using resins such as S-Sepharose FF (Pharmacia), hydrophobic chromatography using resins such as butyl Sepharose and phenyl Sepharose, gel filtration using a molecular sieve, affinity chromatography, chromatofocusing, and electrophoresis such as isoelectric focusing, alone or in combination.

When the protein is expressed in cells in an insoluble form, the cells are similarly separated and disrupted, followed by centrifugation. The protein is recovered from the precipitate fraction by an ordinary method, and the insoluble form is solubilized with a protein-denaturing agent. The solubilized protein solution is diluted or dialyzed to give a solution containing no protein-denaturing agent or containing the protein-denaturing agent at such a low concentration that denaturation of protein is not caused, and the normal protein structure is restored, followed by the same isolation and purification steps as mentioned above to obtain a purified protein preparation.

When the protein of the present invention or its derivative such as sugar-modified form is extracellularly secreted, the protein or its derivative such as sugar chain-added form can be recovered from the culture supernatant. That is, the culture is treated by the above-described means such as centrifugation, and the obtained soluble fraction is subjected to the same isolation and purification steps as mentioned above to obtain a purified protein preparation.

The protein of the present invention can also be produced by chemical synthetic methods such as the Fmoc method (the fluorenylmethyloxycarbonyl method) and the tBoc method (the t-butyloxycarbonyl method). Further, the protein can be chemically synthesized by using peptide synthesizers (Advanced ChemTech, Perkin Elmer, Pharmacia, Protein Technology Instrument, Synthecell-Vega, PerSeptive, Shimadzu Corporation, etc.).

The structure analysis of the purified protein of the present invention can be carried out by methods conventionally used in protein chemistry, e.g. the method described in Hisashi Hirano, Protein Structure Analysis for Gene Cloning, Tokyo Kagaku Dojin (1993).

The protein of the present invention activates eosinophils to cause release of various mediators having cell-killing activity or parasiticidal activity, such as proteins in eosinophil granules and active oxygen, from eosinophils and accumulation of eosinophils. Therefore, the protein can be used as a therapeutic agent for parasitism caused by filaria, blood fluke, lung fluke, hookworm, trichina worm, lung nematode, Gnathostoma, tapeworm, roundworm, etc. and malignant tumors such as solid carcinoma.

The protein of the present invention can be administered as such, as a therapeutic agent, but it is usually preferred to provide pharmaceuticals comprising the protein in the form of pharmaceutical compositions prepared by mixing the protein with one or more pharmaceutically acceptable carriers according to methods well known in the technical field of pharmaceutics. Specifically, sterile solutions in aqueous carriers such as water and aqueous solutions of sodium chloride, glycine, glucose, human albumin, etc. are preferred. Pharmaceutically acceptable additives such as buffer agents and isotonizing agents for adapting the solutions to physiological conditions, e.g. sodium acetate, sodium chloride, sodium lactate, potassium chloride and sodium citrate, may be added. It is also possible to freeze-dry the compositions for storage and dissolve them in an appropriate solvent before each use.

It is preferred to employ the administration route which is the most effective for the treatment. Usually, administration is made non-orally, for example, by subcutaneous, intramuscular or intravenous administration, or administration through respiratory tract.

The protein of the present invention can be administered as such, as a therapeutic agent, but it is usually preferred to provide therapeutic agents comprising the protein in the form of pharmaceutical compositions prepared by mixing the protein with one or more pharmaceutically acceptable carriers according to methods well known in the technical field of pharmaceutics.

It is preferred to employ the administration route which is the most effective for the treatment. For example, administration is made orally, or non-orally by intraoral, intrarectal, subcutaneous, intramuscular or intravenous administration or administration through respiratory tract. Examples of the forms for administration are sprays, capsules, tablets, granules, syrups, emulsions, suppositories, injections, ointments and tapes.

Examples of the compositions suitable for oral administration are emulsions, syrups, capsules, tablets, powders and granules. Liquid compositions such as emulsions and syrups can be prepared using, as additives, water, sugars such as sucrose, sorbitol and fructose, glycols such as polyethylene glycol and propylene glycol, oils such as sesame oil, olive oil and soybean oil, preservatives such as p-hydroxybenzoates, flavors such as strawberry flavor and peppermint, etc. Capsules, tablets, powders and granules can be prepared using, as additives, excipients such as lactose, glucose, sucrose and mannitol, disintegrating agents such as starch and sodium alginate, lubricants such as magnesium stearate and talc, binders such as polyvinyl alcohol, hydroxypropyl cellulose and gelatin, surfactants such as fatty acid esters, plasticizers such as glycerin, etc.

Examples of the compositions suitable for non-oral administration are injections, suppositories and sprays. Injections are prepared using a carrier which comprises a salt solution, a glucose solution, or a mixture of a salt solution and a glucose solution. Suppositories are prepared using a carrier such as cacao fat, hydrogenated fat or carboxylic acid. The active compound can be administered as such in the form of spray, but sprays may be prepared using a carrier which does not irritate oral mucosa or mucosa of respiratory tract and which is suitable for dispersing the compound as fine particles to facilitate absorption thereof, e.g. lactose and glycerin. Sprays can be prepared in the form of aerosol, dry powder, etc. according to the properties of the active compound and carrier used. In preparing these compositions for non-oral administration, the above-mentioned additives for the compositions for oral administration may also be added.

The dose and administration schedule will vary depending on the desired therapeutic effect, the administration route, the period of treatment, the patient's age and body weight, etc. However, an appropriate daily dose for an adult is generally 10 µg/kg to 8 mg/kg.

The cell which is specifically acted on by the protein of the present invention or the cell membrane or the receptor which specifically binds to the protein of the present invention can be detected or obtained by bringing said protein into contact with a test sample by using methods such as intracellular calcium concentration measurement, chemotaxis assay and receptor binding assay.

Examples of the test samples are cells or tissues which are specifically acted on by the protein of the present invention and cell membranes or receptors which specifically bind to the protein of the present invention.

Examples of the cells which are specifically acted on by the protein of the present invention are leukocytes such as lymphocytes (e.g. B cells, T cells and large granular lymphocytes), phagocytes (e.g. mononuclear phagocytes, neutrophils and eosinophils) and accessory cells (e.g. basophils, mast cells and platelets), among which eosinophils are preferably employed. In the present invention, the cells may be in any form, for example, a single cell, a cell aggregate or a tissue. Examples of the cell membranes or receptors which specifically bind to the protein of the present invention are cell membranes prepared from the above cells and receptors isolated and purified from said cells.

Procedures of the intracellular calcium concentration measurement, the chemotaxis assay and the receptor binding assay are described below.

### [Intracellular Calcium Concentration Measurement]

After incorporation of a fluorescence calcium indicator (e.g. fura-2) into a test sample (e.g. eosinophils), the protein of the present invention is added to the sample. The fluorescence intensity is continuously measured before and after the addition of the protein to detect the change in intracellular calcium concentration. The cells showing temporary increase in intracellular calcium concentration are judged to be acted on by the protein of the present invention.

### [Chemotaxis Assay]

Boyden's chamber containing a test sample (e.g. eosinophils) in the upper chamber above the membrane filter and a solution of the protein of the present invention in the lower chamber is allowed to stand for an appropriate time, and the number of cells which moved to the lower chamber through the filter is counted. If a large number of cells moved to the lower chamber compared with the case in which a buffer without the protein of the present invention is used, it is judged that the protein of the present invention has chemotactic action on the cells.

### [Receptor Binding Assay]

The protein of the present invention is labeled with a radioisotope or the like by, for example, attaching ¹²⁵I to Tyr residue of the protein by using Bolton-Hunter reagent. The labeled protein and the membrane fraction prepared from cells are subjected to reaction by mixing at 4 to 37°C for 20 minutes to 24 hours. After the reaction, the mixture is filtered through a glass filter, followed by washing. The radioactivity of the membrane fraction isolated on the filter is determined to obtain the amount of total binding. Separately, the membrane fraction is subjected to reaction with the labeled protein and a large excess of an unlabeled protein using the same reaction system as above, and the radioactivity of the resulting mixture is determined to obtain the amount of nonspecific binding. The amount of specific binding of receptors to the protein of the present invention can be calculated by subtracting the amount of nonspecific binding from that of total binding. The cells showing specific binding of receptors to the protein of the present invention in this assay system are judged to have the receptors which specifically bind to the protein of the present invention.

The agonist or the antagonist can be detected or obtained by comparing the result obtained by bringing the protein of the present invention into contact with the cell, the cell membrane or the receptor obtained by the above method with the result obtained by bringing the protein of the present invention into contact with the cell, the cell membrane or the receptor obtained by the above method and a test compound.

Examples of the test compounds are low molecular weight compounds, peptides, proteins and antibodies.

In a system using intracellular calcium concentration measurement, the calcium concentration is measured in the presence of a test compound, and a substance which makes the increase in calcium concentration less as compared with that measured in the presence of only the protein of the present invention and the buffer is selected as the antagonist.

In a system using receptor binding assay, the amount of specific binding of the receptor which specifically binds to the protein of the present invention is measured in the presence of a test compound, and a substance the addition of which results in decrease in the amount of specific binding is selected as a substance binding to the receptor. When a test compound alone is added instead of the protein of the present invention in this system, a compound showing the action similar to that of the protein of the present invention is judged to be the agonist, and a compound showing the antagonistic action is judged to be the antagonist.

The agonist of the present invention is capable of activating eosinophils as well as the protein of the present invention, and thus is useful as a therapeutic agent for parasitism, malignant tumors, etc.

The antagonist of the present invention acts antagonistically against the protein of the present invention, that is, suppresses the infiltration of eosinophils activated by the protein of the present invention. Therefore, it is useful as a therapeutic agent for diseases associated with eosinophils infiltration, for example, allergic inflammatory diseases such as asthma, allergic conjunctivitis, allergic rhinitis, atopic dermatitis and allergic bronchopulmonary aspergillosis, eosinophilic pneumonia, and hypereosinophilic syndrome. When the antagonist of the present invention is an antagonist of a chemokine protein, it can be used as a therapeutic agent for autoimmune diseases such as autoimmune hemolytic anemia, primary biliary cirrhosis and systemic lupus erythematosus.

The pharmaceutical compositions comprising the agonist or the antagonist of the present invention can be prepared and administered in the same manner as in the preparation and administration of the pharmaceutical compositions comprising the protein of the present invention except that the agonist or the antagonist of the present invention is used in place of the protein of the present invention.

The antibodies of the present invention include any polyclonal antibodies and monoclonal antibodies which are capable of specific binding to the protein of the present invention.

The polyclonal antibody can be prepared by separation and purification from a serum obtained from an animal immunized with an antigen. For the preparation of the monoclonal antibody, an antibody-forming cell obtained form an animal immunized with an antigen and a myeloma cell are fused to obtain hybridoma, and the hybridoma is cultured or administered to an animal to cause ascites tumor. The monoclonal antibody can be prepared by separation and purification from the resulting culture or ascites.

The antigen can be prepared by separating and purifying the protein of the present invention from various cultured human-derived cells, or by introducing the recombinant vector of the present invention into a non-human host such as E. coli, yeast, an animal cell or an insect cell and then separating and purifying the protein of the present invention produced as an expression product. The antigen can also be prepared by synthesizing a polypeptide having a partial sequence of the protein of the present invention by using an amino acid synthesizer.

Immunization can be carried out by administering the antigen subcutaneously, intravenously or intraperitoneally to a non-human mammal such as rabbit, goat, or 3 to 20-weeks-old rat, mouse or hamster. It is preferred to administer the antigen in combination with a carrier protein with high antigenecity such as Macroschisma hemocyanin, keyhole limpet hemocyanin, bovine serum albumin or bovine thyroglobulin, or an appropriate adjuvant such as complete Freund's adjuvant, aluminum hydroxide gel or pertussis vaccine.

Administration of the antigen is repeated 3 to 10 times after the first administration at intervals to 1 to 2 weeks. On the third to seventh day after each administration, a blood sample is collected from fundus oculi veniplex and the obtained serum is examined for reactivity to the antigen used for immunization by measuring the antibody titer according to enzyme immunoassay [Antibodies - A Laboratory Manual, Cold Spring Harbor Laboratory (1988)]. A non-human mammal whose serum shows a sufficient antibody titer against the antigen used for immunization is employed as a source of serum or antibody-forming cell.

The polyclonal antibody can be prepared by separation and purification from the serum.

For the preparation of the monoclonal antibody, the antibody-forming cell and a myeloma cell derived from a non-human mammal are fused to obtain hybridoma, and the hybridoma is cultured or administered to an animal to cause ascites tumor. The monoclonal antibody can be prepared by separation and purification from the resulting culture or ascites.

Examples of the antibody-forming cells are spleen cells and antibody-forming cells in lymph nodes or peripheral blood, among which spleen cells are preferably used.

As the myeloma cells, mouse-derived cell strains are preferably used. Examples of suitable cell strains are P3-X63Ag8-U1(P3-U1) strain [Current Topics in Microbiology and Immunology, 18, 1-7 (1978)], which is 8-azaguanine-resistant mouse (BALB/c-derived) myeloma cell strain, P3-NS1/1-Ag41(NS-1) strain [European J. Immunology, 6, 511-519 (1976)], SP2/0-Ag14 (SP-2) strain [Nature, 276, 269-270 (1978)], P3-X63-Ag8653(653) strain [J. Immunology, 123, 1548-1550 (1979)] and P3-X63-Ag8(X63) strain [Nature, 256, 495-497 (1975)].

The hybridoma cells can be prepared in the following manner.

The antibody-forming cells and themyeloma cells are mixed and suspended in HAT medium (a medium prepared by adding hypoxanthine, thymidine and aminopterin to a normal medium), followed by culturing for 7-14 days. After the culturing, a portion of the culture supernatant is subjected to enzyme immunoassay to select cells which react with the antigen and do not react with the protein containing no antigen. Then, cloning is carried out by limiting dilution, and cells showing a high and stable antibody titer according to enzyme immunoassay are selected as the monoclonal antibody-forming hybridoma cells.

The monoclonal antibody can be prepared by separation and purification from the culture of the hybridoma cells or from the ascites of an animal having ascites tumor caused by intraperitoneal administration of the hybridoma cells.

Separation and purification of the polyclonal antibody and the monoclonal antibody can be carried out by using means such as centrifugation, ammonium sulfate precipitation, caprylic acid precipitation, and chromatography using DEAE-Sepharose column, anion exchange column, protein A or G-column or gel filtration column, alone or in combination.

The antibody of the present invention reacts specifically with the protein of the present invention, and suppresses the infiltration of eosinophils activated by the protein of the present invention. Therefore, it is useful as a therapeutic agent for diseases associated with eosinophils infiltration, for example, allergic inflammatory diseases, eosinophilic pneumonia and hypereosinophilic syndrome. When the antibody of the present invention reacts specifically with a chemokine protein, it can be used as a therapeutic agent for autoimmune diseases. The pharmaceutical compositions comprising the antibody of the present invention can be prepared and administered in the same manner as in the preparation and administration of the pharmaceutical compositions comprising the protein of the present invention except that the antibody of the present invention is used in place of the protein of the present invention.

By the use of the antibody of the present invention, the protein of the present invention can be immunologically detected or determined.

Immunological detection of the protein can be carried out by ELISA using microtiter plate, fluorescent antibody technique, western blot technique, immune tissue staining technique, etc.

Immunological determination of the protein can be carried out by sandwich ELISA using two kinds of monoclonal antibodies recognizing different epitopes among antibodies reacting with the protein of the present invention in a liquid phase, radioimmunoassay using the protein of the present invention labeled with a radioisotope such as ¹²⁵I and the antibody recognizing the protein of the present invention, etc. Further, immune tissue staining technique using a pathological tissue piece is also applicable.

The antibody of the present invention can be used in the diagnosis of allergic inflammatory diseases, eosinophilic pneumonia, hypereosinophilic syndrome and autoimmune diseases, in which cell or tissue samples taken from a healthy subject and a suspected subject are subjected to immunological detection or determination of the protein of the present invention using the antibody of the present invention to see whether the expression of the protein in cells or tissues of the suspected subject is increased as compared with that of the healthy subject. The antibody of the present invention can also be used as a diagnostic agent for allergic inflammatory diseases, eosinophilic pneumonia, hypereosinophilic syndrome and autoimmune diseases.

The sense DNA and the antisense DNA of the present invention can be used as a probe for northern blot hybridization, southern blot hybridization, *in situ* hybridization, etc., or as a primer for PCR, RT-PCR, etc. In these procedures, the sense DNA and the antisense DNA may be used as such or in the form of an oligonucleotide comprising a part of the nucleotide sequence thereof.

Examples of the oligonucleotides comprising a part of the nucleotide sequence of the present invention are oligonucleotides having a nucleotide sequence consisting of continuous 5-100 residues, preferably 10-50 residues, in the sequence of the DNA of the present invention, for example, the DNA having the nucleotide sequence represented by SEQ ID NO: 12.

The oligonucleotides include DNA, RNA and their derivatives such as methylated forms and phosphorothioate forms.

The mRNA coding for the protein of the present invention can be detected or determined by using the sense DNA or the antisense DNA of the present invention or the oligonucleotide comprising a part of the nucleotide sequence thereof according to northern blot hybridization, RT-PCR, etc.

Detection of the mRNA coding for a chemokine protein by the use of the oligonucleotide comprising a part of the nucleotide sequence of the DNA of the present invention can be carried out by northern hybridization [Molecular Cloning, A Laboratory Manual, Second Edition, Cold Spring Harbor Laboratory Press (1989)], PCR [PCR Protocols, Academic Press (1990)], RT (Reverse Transcription)-PCR [PCR Protocols, Academic Press (1990)], etc. RT-PCR is a method which comprises converting RNA isolated from a tissue or a cell into cDNA by using oligo(dT) primer and reverse transcriptase, subjecting the cDNA to PCR using as primers a pair of oligonucleotides corresponding to the mRNA to be detected, and detecting the amplified fragment. RT-PCR is simpler than northern hybridization and thus is suitable for use in diagnosis of diseases.

Suitable oligonucleotide primers include a sense primer corresponding to the 5' sequence in the nucleotide sequence of the mRNA to be detected and an antisense primer corresponding to the 3' sequence therein. Thymidine in an oligonucleotide primer corresponds to uracil in mRNA.

It is preferred to use as the sense primer and the antisense primer oligonucleotides which are not greatly different from each other in melting temperature (Tm) and number of bases. Preferred number of bases is 5-100, particularly 10-50.

Any partial nucleotide sequence of mRNA can be used as the partial nucleotide sequence to be amplified using the oligonucleotide primers. However, it is preferred to use partial sequences of 50 bp to 2 kbp which do not contain a repeated sequence or G-C (guanine-cytosine)-rich sequence.

The antisense DNA of the present invention [Chemistry, 46, 681 (1991), Biotechnology, 9, 358 (1992)] can also be used for the treatment of autoimmune diseases by repressing DNA transcription or mRNA translation.

Repression of chemokine protein production by using antisense DNA technique can be carried out by administering to an living organism an oligonucleotide designed and prepared based on a partial nucleotide sequence of the DNA coding for the chemokine protein of the present invention, preferably, a sequence of 10-50 bases in the translation initiation region. As the nucleotide sequence of the synthetic oligonucleotide, sequences which agree with a part of the nucleotide sequence of the antisense DNA chain of the present invention and modifications thereof which retain the activity to inhibit the expression the protein activity can be used.

The sense DNA or the antisense DNA of the present invention or the oligonucleotide comprising a part of the nucleotide sequence thereof can be used in the diagnosis of allergic inflammatory diseases, eosinophilic pneumonia, hypereosinophilic syndrome, autoimmune diseases, malignant tumors and parasitism. That is, cell or tissue samples taken from a healthy subject and a suspected subject are subjected to detection or determination of mRNA coding for the protein of the present invention according to northern hybridization, PCR, etc. using said DNA or oligonucleotide to see whether the expression of the mRNA in cells or tissues of the suspected subject is increased as compared with that of the healthy subject. The sense DNA or the antisense DNA of the present invention or the oligonucleotide comprising a part of the nucleotide sequence thereof can also be used as a diagnostic agent for allergic inflammatory diseases, eosinophilic pneumonia, hypereosinophilic syndrome, autoimmune diseases, malignant tumors and parasitism. Further, the oligonucleotide comprising a part of the nucleotide sequence of the sense DNA or the antisense DNA of the present invention is also useful as a reagent for gene research.

The antisense DNA of the present invention and the oligonucleotide comprising a part of the nucleotide sequence thereof are capable of repressing the transcription of the DNA of the present invention or the mRNA translation, and suppress the infiltration of eosinophils activated by the protein of the present invention. Therefore, they are useful as a therapeutic agent for diseases associated with eosinophils infiltration, for example, allergic inflammatory diseases, eosinophilic pneumonia, hypereosinophilic syndrome and autoimmune diseases. The pharmaceutical compositions comprising the antisense DNA of the present invention or the oligonucleotide comprising a part of the nucleotide sequence thereof can be prepared and administered in the same manner as in the preparation and administration of the pharmaceutical compositions comprising the protein of the present invention except that the antisense DNA of the present invention or the oligonucleotide comprising a part of the nucleotide sequence thereof is used in place of the protein of the present invention.

The sense DNA or the antisense DNA of the present invention or the oligonucleotide comprising a part of the nucleotide sequence thereof can be inserted into virus vectors such as retrovirus and adenovirus or other vectors in the form of single-stranded or double-stranded DNA or oligonucleotide to prepare the vectors for gene therapy.

### Brief Description of the Drawings

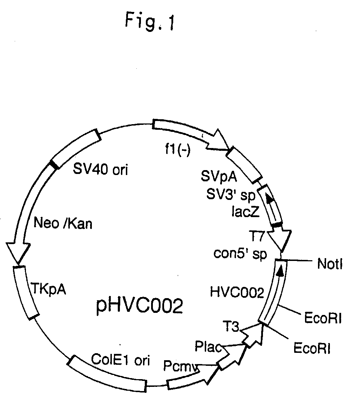

Fig. 1 shows the structure of plasmid pHVC002.

### (Abbreviations in the Drawing)

- f1(-): : replication origin of f1 phage
- SVpA: : poly (A) additional signal of simian virus 40 (SV40) early gene
- SV3'sp: : 3' splicing signal of SV40 early gene
- lacZ: : E. coli β galactosidase gene
- T7: : T7 promoter recognized by T7 phage RNA polymerase
- con5'sp: : consensus 5' splicing signal
- HVC002: : HVC002 cDNA
- T3: : T3 promoter recognized by T3 phage RNA polymerase
- Plac: : promoter of E. coli lactose gene
- Pcmv: : promoter of cytomegalovirus immediate early gene
- ColE1 ori: : replication origin of E. coli Col E1 factor
- TKpA: : poly (A) additional signal of herpes simplex virus thymidine kinase gene
- Neo/Kan: : neomycin/kanamycin resistance gene
- SV40 ori: : replication origin of SV40
- kb: : kilobase pairs
- NotI: : recognition site for restriction enzyme NotI
- EcoRI: : recognition site for restriction enzyme EcoRI

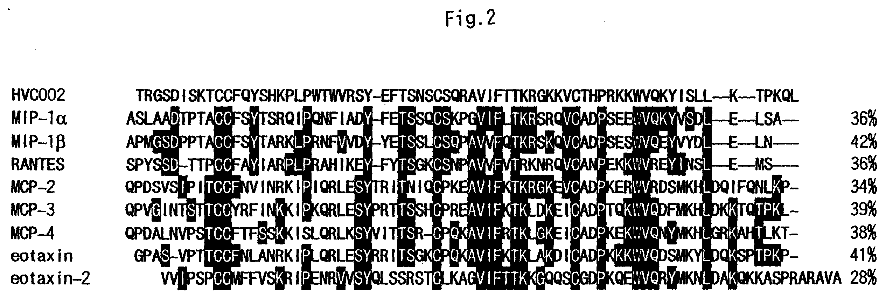

Fig. 2 shows the comparison of the amino acid sequence of the mature protein excluding the signal peptide of the HVC002 protein with those of other human CC chemokines (MIP-1α, MIP-1β, RANTES, MCP-2, MCP-3, MCP-4, eotaxin and eotaxin-2). The amino acid residues of the chemokines which are common with the HVC002 protein are shown in black-and-white reversal. The amino acid sequence homology (%) to the HVC002 protein is shown on the right.

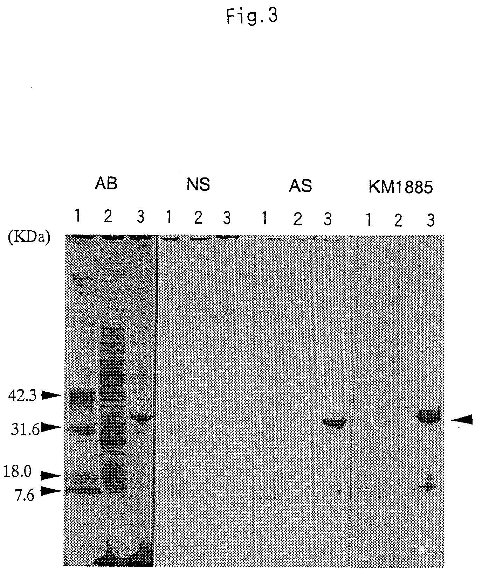

Fig. 3 shows the result of western blotting carried out on thioredoxin-HVC002 fusion protein expressed in E. coli using mouse antiserum and monoclonal antibody KM1885.

### (Abbreviations in the Drawing)

- AB: : Amido Black staining
- NS: : western blotting using mouse normal serum
- AS: : western blotting using mouse antiserum
- KM1885: : western blotting using monoclonal antibody KM1885
- 1: : molecular weight marker
- 2: : 5 µg of E. coli contaminant protein [ultrasonication supernatant of E. coli AD494(DE3)pLysS containing pET32a(+)]
- 3: : 1 µg of partially purified thioredoxin-HVC002 fusion protein
- Arrow: : indicates the location of thioredoxin-HVC002 fusion protein

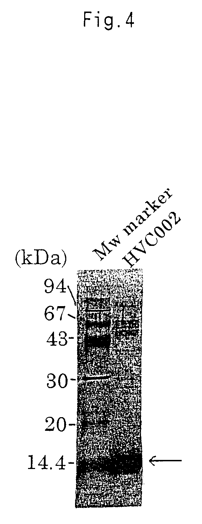

Fig. 4 shows the result of 15% SDS-PAGE carried out on the HVC002 protein expressed in insect cells and purified. The results of electrophoresis, followed by silver staining, on 0.5 ng of the purified HVC002 protein and a molecular weight marker are shown in the right and left lanes, respectively. The arrow indicates the location of the HVC002 protein.

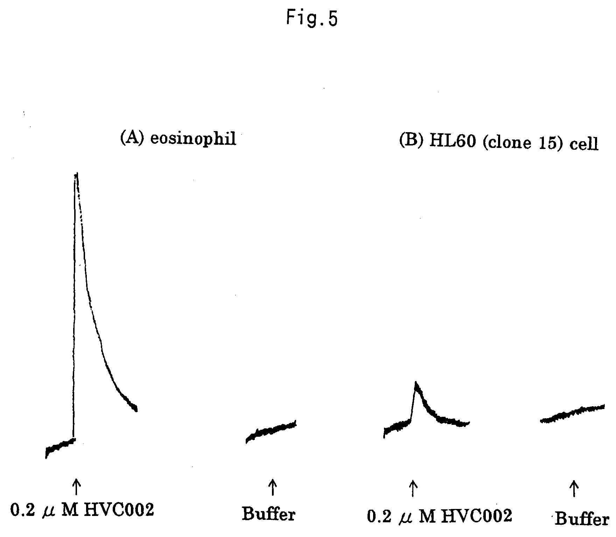

Fig. 5 shows the result of the assay for calcium mobilization of the HVC002 protein on human peripheral eosinophils and HL-60 (clone 15) differentiated into eosinophil-like cells by 0.5 mM butyric acid treatment. (A) shows the result on human peripheral eosinophils and (B) shows the result on HL-60 (clone 15). The numbers on the abscissa indicate the passage of time and those on the ordinate indicate the specific fluorescence intensity (fluorescence intensity with excitation at 340 nm / fluorescence intensity with excitation at 380 nm). The specific fluorescence intensity is proportional to the intracellular calcium concentration. The arrows indicate the points of time when the HVC002 protein and a buffer were added, respectively.

### Best Modes for Carrying Out the Invention

### Example 1 Cloning of a cDNA Fragment of a Novel Gene (HVC002)

### (1) Acquisition of Total RNA from HUVEC

HUVEC (umbilical cord vein vascular endothelial cells, Kurabo Industries Ltd.) were cultured in F-12K medium (Dainippon Pharmaceutical Co., Ltd.) containing heparin sodium, endothelial cell growth supplement (Becton-Dickinson and Co.), 10% fetal calf serum (Biotech International), 1% penicillin (5000 units/ml)·streptomycin (5 mg/ml) solution (Gibco BRL) and 0.15% NaHCO₃ (Sigma) at 37°C in the presence of 5% CO₂. To the medium containing 5 x 10⁶ cells was added 100 units/ml of human IL-4 (Zymogen), and culturing was carried out for 17 hours, followed by collection of cells (hereinafter referred to as IL-4-stimulated HUVEC). To the medium containing 5 x 10⁶ cells was added 10 mg/ml of human TNF-α (Zymogen), and culturing was carried out for 17 hours, followed by collection of cells (hereinafter referred to as TNF-α-stimulated HUVEC). Further, the medium containing 5 x 10⁶ cells was cultured for 17 hours without addition of any stimulating substance and the cells (hereinafter referred to as non-stimulated HUVEC) were collected.

After the collected cells were washed with PBS, total RNA was obtained according to the AGPC method [Experimental Medicine, 9, 1937 (1991)]. That is, the cells were dissolved in 5 ml of D solution (4 M guanidinium thiocyanate, 25mM sodium citrate, 0.5% n-laurylsarcosine, 0.1 M 2-mercaptoethanol), followed by addition of 0.7 ml of 2 M sodium acetate (pH 4.0). To the solution were added 7 ml of water-saturated phenol and 1.4 ml of chloroform-isoamyl alcohol (49:1), and the mixture was vigorously shaken and then allowed to stand on ice for 15 minutes, followed by centrifugation at 4°C at 10,000 x g for 20 minutes. The aqueous layer was mixed with 5.6 ml of isopropanol and the mixture was allowed to stand at -20°C for one hour and then centrifuged at 4°C at 10,000 x g for 20 minutes to precipitate RNA. After the supernatant was removed, 0.5 ml of D solution was added to the precipitate to dissolve the RNA. To the RNA solution was added 0.5 ml of isopropanol, and the mixture was allowed to stand at -20°C for one hour and then centrifuged at 4°C at 10,000 x g for 10 minutes to precipitate the RNA. After the supernatant was removed, the precipitate was washed with 75% ethanol and dried under reduced pressure. Then, the precipitate was dissolved in 300 µl of distilled water and mixed well with 300 µl of 4 M LiCl. The resulting mixture was allowed to stand on ice for one hour and centrifuged at 4°C at 15,000 x g for 10 minutes to precipitate the RNA. After the supernatant was removed, the precipitate was washed with 75% ethanol, dried, and then dissolved in 22 µl of distilled water. By the above procedure, total RNAs were obtained in the following amounts: 36 µg from the IL-4-stimulated HUVEC; 64 µg from the TNF-α-stimulated HUVEC; and 51 µg from the non-stimulated HUVEC.

### (2) Fluorescence Differential Display Using Total RNA of HUVEC

To 2.5 µg of each of the total RNAs of the IL-4-stimulated HUVEC, the TNF-α-stimulated HUVEC and the non-stimulated HUVEC was added distilled water to make a total volume of 9 µl. After addition of 1 µl of anchor primer FAH (nucleotide sequence is shown by SEQ ID NO: 4; Sawady, 50 µM) whose 5' end had been fluorescence-labeled with fluorescein isothiocyanate (hereinafter referred to as FITC), the mixture was heated at 70°C for 5 minutes and then immediately ice-cooled. To the reaction mixture were added 4 µl of 5 x reverse transcriptase reaction buffer [250 mM Tris-HCl (pH 8.3), 275 mM KCl, 15 mM MgCl₂], 2 µl of 100 mM dithiothreitol (DTT), 1 µl of 10 mM dNTP (dATP, dGTP, dTTP and dCTP), 1 µl of distilled water, and 1 µl (200 units) of reverse transcriptase SUPERSCRIPT™ II RNAse H⁻ Reverse Transcriptase (Life Technologies). The resulting mixture was allowed to stand at room temperature for 10 minutes and then subjected to reaction at 42°C for 50 minutes to synthesize cDNA. The reaction was stopped by heating at 90°C for 5 minutes. To the reaction mixture was added 40 µl of TE buffer [10 mM Tris-HCL (pH 8.0), 1 mM disodium ethylenediaminetetraacetate (EDTA) (pH 8.0)] to make a cDNA solution.

To 1 µl of the cDNA solution were added 14.7 µl of distilled water, 2 µl of 10 x PCR buffer [100 mM Tris-HCl (pH 8.8), 500 mM KCl, 15 mM MgCl₂, 1% Triton X-100], 0.8 µl of 2.5 mM dNTP, 0.3 µl of 50 µM fluorescence-labeled anchor primer FAH, 1 µl of primer OPC-10 (5'-TGTCTGGGTG-3'; Operon, 10 µM), and 0.2 µl of DNA polymerase Gene Taq (Nippon Gene, 5 units/µl). The resulting mixture was subjected to reaction using a thermal cycler at 94°C for 3 minutes, at 40°C for 5 minutes and at 72°C for 5 minutes, and subsequently 27 cycles of reactions (one cycle: at 95°C for 15 seconds, at 40°C for 2 minutes and at 72°C for one minute), followed by reaction at 72°C for 5 minutes, to amplify the DNA fragment.

After the completion of reaction, 3 µl of an electrophoresis sample solution (95% formamide, 0.1% xylene cyanole, 0.1% Bromophenol Blue) was added to 4 µl of the reaction mixture. The resulting mixture was heated at 95°C for 2 minutes and then immediately ice-cooled, followed by 6% acrylamide gel electrophoresis using a buffer comprising 89 mM tris(hydroxymethyl)aminomethane, 89 mM boric acid and 2 mM EDTA. After the electrophoresis, the fluorescence intensity of gel was measured by using FluorImager (Molecular Dynamics) to compare the band patterns of the reaction mixtures. As a result, a band of ca. 300 bp was observed only with the amplified cDNA of the IL-4-stimulated HUVEC and was not observed with the amplified cDNA of the non-stimulated HUVEC or that of the TNF-α-stimulated HUVEC. The gel portion corresponding to this band was cut out.

To about one fourth of the gel cut out were added 38 µl of distilled water, 5 µl of 10 x PCR buffer, 4 µl of 2.5 mM dNTP, 0.6 µl of anchor primer NA (nucleotide sequence is shown by SEQ ID NO: 4, non-fluorescence-labeled; Sawady, 34 µM), 2 µl of 10 µM primer OPC-10, and 0.5 µl of DNA polymerase Gene Taq. After heating at 94°C for 3 minutes, the mixture was subjected to 30 cycles of reactions (one cycle: at 95°C for 15 seconds, at 40°C for 2 minutes and at 72°C for one minute), followed by reaction at 72°C for 5 minutes, to amplify the DNA fragment again. After the completion of reaction, the reaction mixture was extracted with phenol-chloroform (1:1) and then with chloroform-isoamyl alcohol (24:1), followed by ethanol precipitation. The precipitate was dissolved and subjected to electrophoresis using 1.5% low-gelling-temperature agarose gel (SEA PLAQUE GTG; FMC BioProducts), followed by ethidium bromide staining. The amplified DNA fragments were cut out and heated at 65°C for 15 minutes to melt agarose. Extraction was carried out with phenol-chloroform and then with chloroform-isoamyl alcohol, followed by ethanol precipitation, and the DNA fragments were dissolved in 10 µl of TE buffer.

The DNA fragment solution (1 µl) was mixed with 1 µl of pT7Blue T-Vector (Novagen, vector for PCR fragment cloning) and subjected to reaction for inserting the DNA fragment into the plasmid using DNA ligation kit ver. 1 according to the instructions attached thereto(Takara Shuzo Co., Ltd.). Escherichia coli DH5α (Life Technologies) was transformed to obtain an ampicillin-resistant strain, and a plasmid DNA was isolated from the obtained transformant by a known method. The plasmid DNA (0.3 ng) was dissolved in 19 µl of distilled water, and 2.5 µl of 10 x PCR buffer, 2 µl of 2.5 mM dNTP, 0.3 µl of 34 µM anchor primer NA, 1 µl of 10 µM primer OPC-10 and 0.5 µl of DNA polymerase Gene Taq were added thereto. The mixture was subjected to reaction for amplification of DNA fragment in the same manner as in the above second amplification of DNA fragment. As a result, a fragment of ca. 300 bp was amplified, whereby it was confirmed that the amplified DNA fragment was inserted into said plasmid.

The nucleotide sequence of the amplified DNA fragment was determined by using Dye primer cycle sequencing kit (Perkin Elmer) and a DNA sequencer (Perkin Elmer). The reagents and method employed for the sequence determination were those described in the instructions attached to the above kit. The determined nucleotide sequence, which is shown by SEQ ID NO: 3, corresponds to the sequence of bases 150 to 452 in the sequence of SEQ ID NO: 12. The sequence of bases 1 to 10 and the sequence of bases 306 to 322 in the sequence of SEQ ID NO: 3 are derived from the primer OPC-10 and the primer NA, respectively, and therefore the sequence of SEQ ID NO: 12 does not contain the sequences of bases 1 to 3 and 307 to 322 in the sequence of SEQ ID NO: 3.

As a result of the search through the known nucleotide sequences in the GenBank data base, no sequence was found which agreed with the determined sequence.

### Example 2 Detection of Specificity of HVC002 Protein Expression

### (1) Acquisition of mRNA from HUVEC

Human-IL-4-stimulated HUVEC (2 x 10⁷ cells), human-TNF-α-stimulated HUVEC (2 x 10⁷ cells) and non-stimulated HUVEC (2 x 10⁷ cells) were cultured and collected. The culturing and stimulation conditions were the same as in the acquisition of total RNA from HUVEC. By the use of Fast Track mRNA extraction kit (Invitrogen), mRNAs were obtained in the following amounts: 18 µg from the IL-4-stimulated HUVEC; 11 µg from the TNF-α-stimulated HUVEC; and 17 µg from the non-stimulated HUVEC. The reagents and method employed for the mRNA extraction were those described in the instructions attached to the above kit.

### (2) Northern Blot of HVC002

Each of the mRNAs (1 µg) was dissolved in 2.2 µl of distilled water, and 5 µl of formamide, 1.8 µl of 37% formaldehyde solution and 1 µl of 10 x MOPS buffer [0.2 M morpholinopropanesulfonic acid (pH 7.0), 50 mM sodium acetate, 10 mM EDTA (pH 8.0)] were added thereto. The mixture was heated at 65°C for 10 minutes and then ice-cooled, followed by addition of 1 µl of 50% glycerol containing 0.01% Bromophenol Blue. The resulting mixture was subjected to electrophoresis using 1.5% agarose gel containing 2.2 M formaldehyde, and as a buffer, 1 x MOPS (10-fold dilution of 10 x MOPS). After the electrophoresis, the gel was soaked in distilled water for 10 minutes. The distilled water was replaced with fresh one and the gel was further soaked therein for 10 minutes and then in 50 mM NaOH for 20 minutes, followed by replication on nylon membrane filter HybondN⁺ (Amersham) using VacuGene XL Vacuum System (Pharmacia, blotting apparatus). Then, the filter was left on a filter paper soaked in 50 mM NaOH for 5 minutes to fix the RNA, and washed with 2 x SSC [0.3 M NaCl, 30 mM trisodium citrate; n x SSC refers to n-fold concentration of 1 x SSC (0.15 M NaCl, 15 mM trisodium citrate)], followed by air-drying.

Separately, 0.6 ng of the plasmid carrying the inserted cDNA fragment having the nucleotide sequence of SEQ ID NO: 3 obtained in Example 1 was dissolved in 38 µl of distilled water and subjected to PCR under the same conditions as in the second amplification of the DNA fragment cut out from the gel in Example 1 to amplify the cDNA fragment. The amplified fragment (0.2 µg) was ³²P-labeled using [α-³²P]dCTP and DNA random labeling kit (Boehringer). The reagents and method employed for the labeling were those described in the instructions attached to the above kit. After the labeling, the reaction mixture was subjected to gel filtration using a Sephadex G-50 column and the unreacted [α-³²P]dCTP was removed, whereby a probe was prepared.

The above filter was then sealed in a vinyl bag containing a hybridization solution {6 x SSC, 5 x Denhalt solution [0.1% bovine serum albumin (BSA), 0.1% Ficoll, 0.1% polyvinyl pyrrolidone], 0.5% sodium dodecyl sulfate (SDS), 20 µg/ml denatured DNA obtained by subjecting salmon sperm DNA to ultrasonication, heating in a boiling water bath for 5 minutes, and then rapid cooling in ice}. Prehybridization was carried out by shaking at 65°C for 90 minutes. Then, denatured DNA obtained by subjecting a half of the probe to heating in a boiling water bath for 5 minutes and then rapid cooling in ice was added to the hybridization solution, and the filter was sealed in the vinyl bag containing the resulting solution. Hybridization was carried out by shaking at 65°C for 17 hours.

The filter was taken out of the vinyl bag and washed by shaking in 2 x SSC containing 0.1% SDS at room temperature for 15 minutes. After the solution was replaced with a fresh one, the filter was washed by shaking therein at room temperature for 10 minutes, followed by washing by shaking in 1 x SSC containing 0.1% SDS at 65°C for 30 minutes. The solution was replaced with a fresh one again and the filter was washed by shaking therein at 65°C for 20 minutes. Then, the filter was washed by shaking in 0.1 x SSC containing 0.1% SDS at 65°C for 20 minutes, followed by air-drying. The air-dried filter was subjected to autoradiography at -80°C overnight using a sensitized screen. As a result, a strong signal was detected only with the mRNA of the IL-4-stimulated HUVEC and a signal was not detected with the mRNA of the non-stimulated HUVEC or that of the TNF-α-stimulated HUVEC. The size of the mRNA was about 0.7 kb.

### Example 3 Cloning of HVC002 cDNA

### (1) Preparation of cDNA Library

Distilled water was added to 5 µg of the mRNA of the IL-4-stimulated HUVEC obtained in Example 2 and 2.5 µg of NotI-primer-adapter (Promega) to make up to 7 µl, followed by heating at 70°C for 10 minutes and rapid cooling in ice. To the mixture were added 4 µl of 5 x reverse transcriptase reaction buffer, 2 µl of 100 mM DTT, 1 µl of 10 mM dNTP, and as a tracer, 1 µl of [α-³²P]dCTP (110 TBq/mmol, Amersham), followed by heating at 37°C for 2 minutes. After addition of 5 µl (1000 units) of reverse transcriptase SUPERSCRIPT™ II RNAse H⁻ Reverse Transcriptase, the mixture was subjected to reaction at 44°C for one hour to synthesize cDNA. To the reaction mixture were added 82 µl of distilled water, 32 µl of 5 x reaction buffer [100 mM Tris-HCl, 500 mM KCl, 25 mM MgCl₂, 50 mM (NH₄)₂SO₄, 10 mM DTT, 250 mg/ml BSA, 750 mM β-nicotinamide dinucleotide], 2.75 µl of 10 mM dNTP, 2.75 µl of [α-³²P]dCTP, 5.5 µl of 100 mM DTT, 2.5 µl of 6 units/µl E. coli DNA ligase (Takara Shuzo Co., Ltd.), 11.5 µl of 3.5 units/µl E. coli DNA polymerase (Takara Shuzo Co., Ltd.) and 2.5 µl of 0.6 unit/µl E. coli ribonuclease H (Takara Shuzo Co., Ltd.). The mixture was subjected to reaction at 16°C for 3 hours to decompose the mRNA and make the cDNA double-stranded. Then, 21 µl of 4 units/µl T4 DNA polymerase (Takara Shuzo Co., Ltd.) was added to the mixture, and reaction was carried out at 16°C for 5 minutes to make blunt ends. The reaction was stopped by addition of 2 µl of 500 mM EDTA (pH 8.0) and 2 ml of 10% SDS, followed by phenol-chloroform extraction to remove the enzymes. In order to remove the cDNA of 400 bp or less and the unreacted NotI-primer-adapter and nucleotide, the aqueous layer was placed in TE buffer-equilibrated SizeSep-400 span column (Pharmacia) and centrifuged at 400 x g for 2 minutes. The eluate was subjected to ethanol precipitation and the cDNA was recovered.

The recovered cDNA was dissolved in 5 µl (50 pmol) of EcoRI adapter (Promega), and 40 µl of solution A of ligation kit Ver. 1 (Takara Shuzo Co., Ltd.) was added thereto, followed by addition of 5 µl of solution B. The mixture was subjected to reaction at 15°C for 2 hours to attach the EcoRI adapter to both ends of the cDNA. After addition of 40 µl of 10 mM EDTA (pH 8.0), the mixture was heated at 65°C for 15 minutes to stop the reaction, followed by ethanol precipitation to recover the cDNA. The cDNA was dissolved in 36 µl of distilled water, and 5 µl of 10 x reaction buffer [500 mM Tris-HCl (pH 7.6), 100 mM MgCl₂], 2.5 µl of 100 mM DTT, 2.5 µl of 10 mM ATP and 4 µl of 6 units/µl T4 polynucleotide kinase (Takara Shuzo Co., Ltd.) were added thereto. The mixture was subjected to reaction at 37°C for 30 minutes to phosphorylate the 5' end of the attached EcoRI adapter. To the reaction mixture were added 7.2 µl of distilled water, 1.8 µl of 5 M NaCl and 8 units (1 µl) of NotI, and the resulting mixture was subjected to reaction at 37°C for 2 hours to cleave the NotI site in the NotI-primer-adapter. After the reaction was stopped by addition of 6 µl of 500 mM EDTA, 1 µl of 20 µg/µl tRNA was added, followed by phenol-chloroform extraction to remove the enzymes. In order to remove the unreacted EcoRI adapter from the obtained aqueous layer, the aqueous layer was placed in TE buffer-equilibrated SizeSep-400 span column and centrifuged at 400 x g for 2 minutes, and the eluate was recovered.

The eluate was ultracentrifuged at 50,000 x g for 3 hours with potassium acetate concentration gradient of 5%-20%, and taken from the bottom in 26 fractions by using a perista pump. Each fraction was subjected to ethanol precipitation and the cDNA was recovered. After the recovered cDNA was subjected to agarose gel electrophoresis, the length of cDNA in each fraction was measured by autoradiography. Fractions containing cDNA of ca. 2 kb or less were combined.

To 9 µg (9 µl) of cloning vector ZAP Express (Stratagene) were added 10 µl of 10 x H restriction enzyme buffer (1 M NaCl, 100 mM Tris-HCl, 10 mM MgCl₂), 75 µl of distilled water and 90 units (6 µl) of EcoRI, and the mixture was subjected to reaction at 37°C for 2 hours. To the reaction mixture were added 1 µl of 5 M NaCl and 40 units (5 µl) of NotI, and reaction was carried out at 37°C for 2 hours. Then, 8 units (1 µl) of NotI was further added, and reaction was carried out at 37°C for one hour to cleave the EcoRI site and the NotI site in the vector. To the reaction mixture were added 100 µl of 2 M Tris-HCl (pH 8.0) and one unit (2 µl) of E. coli C75-derived alkaline phosphatase (Takara Shuzo Co., Ltd.), and reaction was carried out at 60°C for 30 minutes to dephosphorylate the 5' end of the EcoRI cleavage end and the not cleavage end of the vector. After phenol-chloroform extraction was repeated twice to remove the enzymes, chloroform extraction was carried out and the aqueous layer was subjected to ethanol precipitation. The vector DNA obtained as a precipitate was dissolved in TE buffer.

TE buffer containing 1 µg of the vector DNA was added to the cDNA fractions, and the mixture was subjected to ethanol precipitation. The precipitate was dissolved in 4 µl of ligase buffer [100 mM Tris-HCl (pH 7.6), 5 mM MgCl₂, 300 mM NaCl], and 4 µl of solution B of ligation kit Ver. 1 was added thereto. Reaction was carried out at 26°C for 10 minutes to ligate the vector DNA with the cDNA. The reaction mixture was subjected to packaging in 4 µl portions using λ phage packaging kit GigaPack Gold II (Stratagene). The reagents and method employed for the packaging were those described in the instructions attached to the above kit. The obtained phage was used for infection of E. coli XL1-Blue MRF' strain (Stratagene) and the titer was measured. Further, by proliferating the phage on a plate and recovering the same, the cDNA library was amplified once, whereby the desired cDNA library was obtained. The measurement of titer and the amplification of library were carried out according to the instructions attached to the λ phage packaging kit.

### (3) Isolation of HVC002 cDNA Clone

Screening of the cDNA library for the full length cDNA clone was carried out by plaque hybridization. The cDNA library was made to appear in plaques on 20 plates of 15 cm diameter (about 5 x 10⁴ plaques per plate; about 1 x 10⁶ plaques in total), and two nylon membrane filters (HybondN⁺; Amersham) were successively put on each plate (the first filter for one minute and the second filter for 5 minutes), whereby phage DNA on one plate was transferred as a replica to two filters. Each filter was soaked in an alkali treatment solution (0.5 M NaOH, 1.5 M NaCl) for 90 seconds to denature and fix the DNA, and then soaked in a neutralization solution [0.5 M Tris-HCl (pH 7.5), 1.5 M NaCl] for 10 minutes for neutralization. After washing by rubbing with a sponge in 2 x SSC and 0.1% SDS, the filter was soaked in 2 x SSC and 0.1% SDS and heated at 55°C for one hour, followed by washing with 2 x SSC and air-drying.

By using the DNA for probe amplified in Example 2, ³²P-labeled probe was prepared in the same manner as in Example 2. The filter was subjected to prehybridization and hybridization in a vinyl bag in the same manner as in Example 2, except that the prehybridization was carried out for 4 hours and the whole amount of probe was used.

The filter was taken out of the vinyl bag and washed by shaking in 2 x SSC at 65°C for 15 minutes. After the solution was replaced with a fresh one, this washing step was repeated, followed by washing by shaking in 2 x SSC containing 0.1% SDS at 65°C for 30 minutes. Then, the filter was washed by shaking in 0.1 x SSC containing 0.1% SDS at 65°C for 10 minutes, followed by air-drying. The air-dried filter was attached to a filter paper and subjected to autoradiography at -80°C overnight using a sensitized screen. Plaques which gave exposure signals at the same positions on the screen with two filters replicated from one plate were selected and isolated together with agar around them. The isolated plaques were left overnight in 500 µl of SM buffer [0.1 M NaCl, 0.2% MgCl₂, 50 mM Tris-HCl (pH 7.5), 0.01% gelatin] to elute the phage into SM buffer.

After the obtained solution was diluted 100-fold with SM buffer, 2 µl of the dilution was used for infection of E. coli XL1-Blue MRF', and plaques were formed again on plates of 10 cm diameter. Plaque hybridization was carried out in the same manner as above except that one filter was used for one plate. By selecting positive plaques which were present independently of other plaques, plaques of HVC002 clone were isolated.

The isolated plaques were left overnight in SM buffer to elute the phage into SM buffer. The phage was used together with helper phage ExAssist (Stratagene) for infection of E. coli XL1-Blue MRF' to cleave a part of the phage and release it as a single-stranded DNA phage into the culture supernatant. The single-stranded DNA phage was used for infection of E. coli XLORL in which helper phage can not replicate, and the clone was isolated as a kanamycin-resistant colony. The reagents and method employed for the cleavage were those described in the instructions attached to ZAP Express.

A plasmid DNA was isolated from the clone by a known method. Primer 1 having the sequence of SEQ ID NO: 5 and primer 2 having the sequence of SEQ ID NO: 6 were synthesized based on the nucleotide sequence of the amplified fragment cloned in Example 1. To 1 ng of the plasmid DNA were added 2 µl of 10 x PCR buffer, 0.8 µl of 2.5 mM dNTP, 20 pmol of primer 1 and 20 pmol of primer 2, and distilled water was added thereto to make up to 19.8 µl. After addition of 0.2 µl of DNA polymerase Gene Taq, the mixture was subjected to 30 cycles of PCR (one cycle: at 94°C for one minute, at 50°C for one minute and at 72°C for 2 minutes). As a result, a DNA fragment of ca. 200 bp was amplified, whereby it was confirmed that this clone was the clone of HVC002 gene. The plasmid was named pHVC002. Plasmid pHVC002 was digested with restriction enzymes EcoRI and NotI and then subjected to agarose gel electrophoresis. The size of the cDNA was about 0.5 kb. Escherichia coli XL1-Blue MRF'/pHVC002 carrying pHVC002 was deposited with the National Institute of Bioscience and Human-Technology, Agency of Industrial Science and Technology, 3, Higashi 1-chome, Tsukuba-shi, Ibaraki-ken, Japan (Postal Code 305) on July 17, 1996 as FERM BP-5585.

### (4) Determination of the Nucleotide Sequence of HVC002 cDNA

The nucleotide sequence of HVC002 cDNA in pHVC002 was determined by using Dye primer cycle sequencing FS Ready Reaction Kit (Perkin Elmer) and 77 DNA sequencer (Perkin Elmer). The reagents and method employed for the sequence determination were those described in the instructions attached to the above kit. The determined nucleotide sequence is shown by SEQ ID NO: 12. This sequence contains an open reading frame (ORF) consisting of 94 amino acid residues. As a result of the search through the known amino acid sequences in the data base, no sequence was found which agreed with the amino acid sequence of the ORF, but there was homology between the sequence of the ORF and those of CC chemokines such as MIP-1β, RANTES and MCP-1. It was found that not only the number and positions of Cys residues which are characteristic of CC chemokines, but also all the amino acid residues conserved in these CC chemokines were conserved in the sequence of the HVC002 protein. As shown in Fig. 2, the amino acid sequence of the HVC002 protein showed the highest homology to that of human MIP-1β among the CC chemokines, i.e. 42% homology. The HVC002 protein is considered to be a protein which is extracellularly secreted.

### Example 4 Expression of the HVC002 Protein in E. coli

### (1) Construction of Expression Plasmid pET32a002

From the homology to human MIP-1β protein, it can be presumed that Ala at position 23 (corresponding to the 23rd amino acid) in the sequence of the ORF shown by SEQ ID NO: 2 is the N-terminal amino acid of the mature HVC002 protein. On the basis of this presumption, the HVC002 protein having the amino acid sequence corresponding to the sequence of amino acids 23 to 71 in the sequence of SEQ ID NO: 2 was expressed in E. coli. To 2 µl (40 ng) of pHVC002 were added 10 µl of 10 x PCR buffer, 8 µl of 2.5 mM dNTP, 75.5 µl of distilled water, 2 µl of 10 µM PCR primer PET32002-S (nucleotide sequence is shown by SEQ ID NO: 7; the sequence of bases 9 to 32 in the sequence of SEQ ID NO: 7 corresponds to the sequence of bases 82 to 105 in the sequence of SEQ ID NO: 12), 2 µl of 10 µM PCR primer PET32002-AS (nucleotide sequence is shown by SEQ ID NO: 8; the sequence of bases 13 to 36 in the sequence of SEQ ID NO: 8 corresponds to the sequence of bases 277 to 300 in the sequence of SEQ ID NO: 12), and 0.5 µl of DNA polymerase Gene Taq. After heating at 94°C for one minute, the mixture was subjected to 25 cycles of PCR (one cycle: at 94°C for 30 seconds, at 60°C for one minute and at 72°C for one minute) and then to reaction at 72°C for 10 minutes to amplify a DNA fragment having the sequence of bases 82 to 300 in the sequence of SEQ ID NO: 12. After the completion of reaction, the reaction mixture was extracted with phenol-chloroform (1:1) and then with chloroform-isoamyl alcohol (24:1), followed by ethanol precipitation to purify the reaction product. The reaction product was dissolved in 30 µl of distilled water, and to 20 µl of the solution were added 10 µl of 10 x Bpu1102I reaction buffer [330 mM Tris-acetic acid (pH 7.9), 100 mM magnesium acetate, 5 mM DTT, 660 mM potassium acetate], 10 µl of 0.1% BSA, and 56 µl of distilled water. After addition of 4 µl (16 units) of restriction enzyme Bpu1102I (Takara Shuzo Co., Ltd.), reaction was carried out at 37°C for 2 hours to cleave the 3' region. The reaction mixture was extracted with phenol-chloroform and then with chloroform-isoamyl alcohol, followed by ethanol precipitation to purify the reaction product. The reaction product was dissolved in 78 µl of distilled water, and 10 µl of 10 x NcoI reaction buffer [200 mM Tris-HCl (pH 8.5), 100 mM MgCl₂, 10 mM DTT, 1 M KCl] and 10 µl of 0.1% BSA were added thereto. After addition of 2 µl (20 units) of restriction enzyme NcoI (Takara Shuzo Co., Ltd.), reaction was carried out at 37°C for 10 hours to cleave the 5' region. The reaction mixture was extracted with phenol-chloroform and then with chloroform-isoamyl alcohol, followed by ethanol precipitation to purify the reaction product. The reaction product was subjected to electrophoresis using 1% low-gelling-temperature agarose gel (SEA PLAQUE GTG; FMC BioProducts), followed by ethidium bromide staining. The gel portion containing the stained band was cut out as the portion of amplified DNA fragments, and was heated at 65°C for 15 minutes to melt agarose. Extraction was carried out with phenol-chloroform and then with chloroform-isoamyl alcohol, followed by ethanol precipitation to purify the amplified HVC002 cDNA fragments having the cleaved ends. The obtained fragments were dissolved in 10 µl of TE buffer.

Plasmid vector pET32a (+) (Novagen; 4 µl, 4 µg), which is a vector for the expression of a fusion protein of a foreign protein and E. coli thioredoxin protein in E. coli, was cleaved with restriction enzymes Bpu1102I and NcoI in the same manner as above. Extraction was carried out with phenol-chloroform and then with chloroform-isoamyl alcohol, followed by ethanol precipitation to purify the reaction product. The reaction product was dissolved in 25 µl of distilled water, and 25 µl of 2 M Tris-HCl (pH 8.0) was added thereto. After addition of 1 µl of E. coli alkaline phosphatase (Takara Shuzo Co., Ltd.), reaction was carried out at 56°C for one hour to dephosphorylate the cleaved 5' end of the vector. The reaction mixture was extracted with phenol-chloroform and then with chloroformisoamyl alcohol, followed by ethanol precipitation to purify the reaction product. The vector moiety was purified by low-gelling-temperature agarose gel electrophoresis in the same manner as in the purification of the amplified HVC002 cDNA fragments and was dissolved in 10 µl of TE buffer.

The NcoI-Bpu1102I fragment derived from pET32a(+) (7.5 µl) and the NcoI-Bpu1102I fragment derived from HVC002 (7.5 µl) were subjected to ligation reaction using DNA ligation kit Ver. 1 (Takara Shuzo Co., Ltd.) to insert the amplified HVC002 cDNA fragment into the vector. The reagents and method employed for the ligation were those described in the instructions attached to the above kit. The reaction mixture was used to transform E. coli DH5α, and a transformant was obtained as an ampicillin-resistant strain. A plasmid was isolated from the transformant by the alkali method, whereby E. coli expression plasmid pET32a002 was obtained.

### (2) Expression of Thioredoxin-HVC002 Fusion Protein in E. coli

E. coli AD494(DE3)pLysS (Novagen) was transformed with pET32a002 and the obtained transformant was cultured at 37°C in 10 ml of L medium containing 50 µg/ml ampicillin. When the absorbance of the culture at 600 nm became 1.2, isopropyl galactopyranoside was added to the culture to a concentration of 1 mM to activate the promoter, followed by further culturing at 37°C for 3 hours. The cells were collected by centrifugation at 7000 rpm for 5 minutes. As a control, E. coli AD494(DE3)pLysS transformed with vector pET32a(+) was similarly cultured and the cells were collected. Each cultured cells were dissolved in Remli's sample buffer, followed by heating at 100°C for 5 minutes. Then, the cell solution was subjected to SDS polyacrylamide gel electrophoresis (SDS-PAGE) using 5-20% concentration gradient acrylamide gel PAGEL NPG-520L (Daiichi Pure Chemicals Co., Ltd.) according to the instructions attached thereto. After the electrophoresis, the protein in the acrylamide gel was stained with Coomassie Brilliant Blue As a result, a protein band of ca. 26 kD was detected with E. coli carrying pET32a002, whereas such a band was not detected with E. coli carrying pET32a(+). This band is considered to be thioredoxin-HVC002 fusion protein from its molecular weight.

### (3) Partial Purification of the Thioredoxin-HVC002 Fusion Protein

E. coli transformant obtained by transformation with pET32a002 in (2) above was cultured in 400 ml of L medium in the same manner as in (2) and the cells were collected. The cells were suspended in 30 ml of PBS and disrupted by treatment using ultrasonic disrupter NP200 (Tomy Seiko) at an intensity of 5 for 30 seconds x 10 times. Microscopic observation of the disrupted cell suspension revealed that many particles which were considered to be the inclusion body of thioredoxin-HVC002 fusion protein were present. The cell suspension was then centrifuged at 15000 rpm for 20 minutes. To the precipitate considered to contain the inclusion body was added 10 ml of 4 M urea, and the resulting suspension was centrifuged at 15000 rpm for 20 minutes. The precipitate was separated and suspended in 10 ml of distilled water, followed by centrifugation at 15000 rpm for 20 minutes. The precipitate was separated, whereby partially purified thioredoxin-HVC002 fusion protein was obtained in the form of inclusion body.

The supernatant of the disrupted cell suspension and the washed precipitate were respectively subjected to SDS-PAGE in the same manner as in (2). It was confirmed that the band of ca. 26 kD considered to be thioredoxin-HVC002 fusion protein was present in the precipitate and not in the supernatant.

### Example 5 Preparation of Anti-HVC002 Antibody

### (1) Preparation of Antigen

By the use of peptide synthesizer ACT357 (Advanced Chemtec), peptide HVC002-4 having the sequence shown by SEQ ID NO: 9 corresponding to the sequence of amino acids 73 to 94 in the sequence of SEQ ID NO: 2 was synthesized as the C-terminal peptide of the HVC002 protein. As a control peptide, peptide HVC002-1 having the sequence shown by SEQ ID NO: 10 corresponding to the sequence of amino acids 23 to 33 in the sequence of SEQ ID NO: 2 was similarly synthesized which was supposed to be the N-terminal peptide of the HVC002 protein.

In order to enhance the immunogenicity of peptide HVC002-4, reaction for making covalent bonds was carried out using keyhole limpet hemocyanin (KLH) and N-(m-maleimidobenzoyloxy)succinimide (MBS). That is, KLH (Calbiochem) was dissolved in PBS at a concentration of 10 mg/ml, and 1/10 volume of 25 mg/ml MBS (Nacalai Tesque, Inc.) was added dropwise thereto. Reaction was carried out with stirring for 30 minutes. The reaction mixture was subjected to gel filtration using a column of Sephadex G-25 (Pharmacia) pre-equilibrated with PBS to remove the unreacted MBS and to obtain KLH-MBS. A mixture of 1 mg of peptide HVC002-4 dissolved in 0.1 M sodium phosphate buffer (pH 7.0) and 2.5 mg of KLH-MBS was subjected to reaction at room temperature for 3 hours with stirring. After the completion of reaction, the mixture was dialyzed against PBS containing 0.5 M NaCl to obtain HVC002-4/KLH conjugate to be used as immunogen.

### (2) Immunization of Animals

The conjugate of HVC002-4, which is the C-terminal peptide of the HVC002 protein, with KLH (100 µg) prepared by the method described in (1) was administered to a 5-weeks-old female Balb/c mouse together with 2 mg of aluminum gel and 1 x 10⁹ cells of pertussis vaccine (Serum Institute of Chiba Prefecture). From two weeks after this administration, 100 µg of the HVC002-4/KLH conjugate was administered once a week for four weeks.

### (3) Measurement of Serum Antibody Titer by Enzyme Immunoassay

Blood samples were collected from fundus oculi veniplex of the immunized mice, and the serum antibody titer was measured by enzyme immunoassay as shown below.

HVC002-4/thyroglobulin (THY) conjugate was obtained by covalently bonding HVC002-4 to THY in the same manner as in (1) except that THY and succinimidyl 4-(N-maleimidomethyl)cyclohexane-1-carboxylate (SMCC) were used instead of KLH and MBS. The HVC002-4/THY conjugate (10 µg/ml) and HVC002-1/THY conjugate obtained by covalently bonding control peptide HVC002-1 to THY (10 µg/ml) were respectively put into wells of a 96-well EIA plate (Gliner) in an amount of 50 µl/well. The plate was allowed to stand at 4°C overnight to adsorb the antigens thereto. After washing with PBS, PBS containing 1% BSA (BSA-PBS) was added in an amount of 100 µl/well, and the plate was allowed to stand at room temperature for one hour to block the remaining active groups. After BSA-PBS was removed, the antiserum of immunized mouse was added in an amount of 50 µl/well and the plate was allowed to stand at room temperature for 2 hours. Then, the plate was washed with PBS containing 0.05% Tween 20 (Tween-PBS), and horseradish peroxidase (HRP)-labeled rabbit anti-mouse immunoglobulin antibody (Daco) was added as the second antibody in an amount of 50 µl/well. The plate was allowed to stand at room temperature for one hour, followed by washing with Tween-PBS. Color was developed by the addition of ABTS substrate solution [a solution prepared by dissolving 550 mg of 2,2'-azinobis(3-ethylbenzothiazoline-6-sulfonic acid), diammonium salt in l L of 0.1 M citrate buffer (pH 4.2) and adding thereto, immediately before use, 1 µl/ml hydrogen peroxide], and the absorbance at 415 nm was measured by using plate reader NJ2001 (InterMed Japan). Mice whose sera showed a clear color development with HVC002-4 and showed no color development with HVC002-1 or BSA, that is, mice showing a sufficient antibody titer were selected.

### (4) Examination of the Specificity of the Antiserum by Western Blotting

The polyclonal antibody in the anti-HVC002 antiserum selected in (3) was examined for reaction specificity according to Western blotting. That is, 1 µg of the thioredoxin-HVC002 fusion protein expressed in E. coli and partially purified in Example 4 and 5 µg of E. coli contaminant protein containing thioredoxin protein as a control (a supernatant obtained by subjecting E. coli AD494(DE3)pLysS carrying pET32a(+) to ultrasonic disruption and then centrifugation) were subjected to SDS-PAGE in the same manner as in Example 4. After the electrophoresis, the proteins in the gel were transferred onto a poly(vinylidene fluoride)(PVDF) membrane by a known blotting method [Antibodies - A Laboratory Manual, Cold Spring Harbor Laboratory (1988)]. After blocking of the PVDF membrane with 1% BSA solution, the membrane was subjected to reaction with the anti-HVC002 antiserum at room temperature for 2 hours. Then, the membrane was washed well with PBS-Tween and was subjected to reaction with the HRP-labeled anti-mouse immunoglobulin antibody at room temperature for one hour. After the PVDF membrane was washed well, color was developed by using HRP-color development reagent (Bio Rad). The reagents and method employed for the color development were those described in the instructions attached to the above reagent. As a result, it was confirmed that the anti-HVC002 antiserum reacted only with the thioredoxin-HVC002 fusion protein of ca. 26 kD and not with the thioredoxin protein or the E. coli contaminant protein. In order to detect the whole protein blotted, electrophoresis and blotting were carried out in the same manner as above, followed by Amido Black staining. The result is shown in Fig. 3.

### (5) Preparation of Monoclonal Antibody-Forming Hybridoma

In order to obtain antibody-forming cells, the spleen was excised from a mouse showing a sufficient antibody titer selected in (3) three days after the last immunization. The spleen was cut into small pieces in MEM medium (Nissui Pharmaceutical Co., Ltd.) and the pieces were loosened with tweezers, followed by centrifugation (1200 rpm, 5 minutes). The supernatant was discarded and the precipitate was treated with Tris-ammonium chloride buffer (pH 7.65) for 1-2 minutes to remove erythrocytes. Washing with MEM medium was carried out three times to obtain mouse spleen cells.

As the parent strain for cell fusion, 8-azaguanine-resistant mouse (BALB/C-derived) myeloma cell strain P3-X63Ag8-U1 (P3-U1) [Current Topics in Microbiology and Immunology, 18, 1 (1978)] was cultured in a normal medium [a medium prepared by adding 1.5 mM glutamine, 50 µM 2-mercaptoethanol, 10 µg/ml gentamicin and 10% fetal calf serum (FCS) to RPMI-1640 medium] to obtain more than 2 x 10⁷ cells of mouse myeloma cells.

The mouse spleen cells and the mouse myeloma cells were mixed at a ratio of 10:1 and centrifuged at 1200 rpm for 5 minutes. The supernatant was discarded and the precipitated cells were loosened well, followed by addition of a cell fusion medium (a medium prepared by adding 2 g of polyethylene glycol #1,000 and 0.7 ml of dimethyl sulfoxide to 2 ml of MEM medium) in an amount of 0.2-1 ml for 10⁸ mouse spleen cells at 37°C with stirring. After 1-2 ml of MEM medium was added several times at intervals of 1-2 minutes, MEM medium was further added to make a total volume of 50 ml, followed by centrifugation at 9000 rpm for 5 minutes. The supernatant was discarded and the precipitated cells were gently loosened. To the cells was added 100 ml of HAT medium (a medium prepared by adding 100 µM hypoxanthine, 15 µM thymidine and 400 nM aminopterin to a normal medium), and suction and discharge were gently repeated using a measuring pipette to suspend the cells in the medium. The cell suspension was put into wells of a 96-well plate in an amount of 100 µl/well, and cultured in a 5% CO₂ incubator at 37°C for 10-14 days. The culture supernatant was examined by enzyme immunoassay as described in (3) to select cells which reacted with antigen peptide HVC002-4 and the thioredoxin-HVC002 fusion protein and did not react with control peptide HVC002-1 or E. coli contaminant protein containing no HVC002 protein. Cloning of the selected cells was carried out twice by limiting dilution [first cloning: HT medium (a medium having the composition of HAT medium excluding aminopterin), second cloning: normal medium], and a clone showing a high and stable antibody titer was selected as the anti-HVC002 monoclonal antibody-forming hybridoma strain, whereby hybridoma KM1885 was established. Hybridoma KM1885 was deposited with the National Institute of Bioscience and Human-Technology, Agency of Industrial Science and Technology, 3, Higashi 1-chome, Tsukuba-shi, Ibaraki-ken, Japan (Postal Code 305) on November 20, 1997 as FERM BP-6177.

### (6) Examination of the Specificity of the Monoclonal

### Antibody by Western Blotting

The anti-HVC002 monoclonal antibody selected in (5) was examined for reaction specificity according to Western blotting. In the same manner as in (4), the thioredoxin-HVC002 fusion protein expressed in E. coli and the control protein were subjected to electrophoresis and transferred onto a PVDF membrane by blotting, followed by reaction with the anti-HVC002 monoclonal antibody KM1885 instead of the antiserum. As shown in Fig. 3, it was confirmed that the anti-HVC002 monoclonal antibody KM1885 reacted only with the thioredoxin-HVC002 fusion protein of ca. 26 kD and not with the thioredoxin protein or the E. coli contaminant protein.

### Example 6 Expression of the HVC002 Protein in Insect Cells

### (1) Construction of a Recombinant Virus for the Expression of the HVC002 Protein in Insect Cells

For the production of a protein in insect cells, it is necessary to construct a recombinant virus carrying the desired gene. The construction process comprises the step of inserting cDNA coding for the desired protein into a plasmid called a transfer vector and the step of obtaining a recombinant virus through homologous recombination caused by cotransfection of a wild-type virus DNA and the transfer vector in an insect cell. These steps were carried out using BaculoGold Starter Kit (Pharmingen) according to the instructions attached thereto as described below.

Plasmid pHVC002 (3 µg) was added to 30 µl of a buffer [50 mM Tris-HCl (pH 7.5), 10 mM MgCl₂, 100 mM NaCl, 1 mM DTT], and 10 units of restriction enzyme PstI was added thereto. The mixture was subjected to reaction at 37°C for 4 hours, followed by ethanol precipitation. The obtained precipitate was dissolved in 30 µl of a buffer [20 mM Tris-HCl (pH 8.5), 10 mM MgCl₂, 100 mM KCl, 1 mM DTT], and 10 units of BamHI was added thereto. The mixture was subjected to reaction at 37°C for 4 hours. The reaction mixture was subjected to agarose gel electrophoresis, and about 0.3 µg of a DNA fragment of 376 bp containing the cDNA coding for HVC002 was obtained.

Then, 3 µg of transfer vector plasmid pVL1393 contained in BaculoGold Starter Kit was added to 30 µl of a buffer [50 mM Tris-HCl (pH 7.5), 10 mM MgCl₂, 100 mM NaCl, 1 mM DTT], and 10 units of PstI was added thereto. The mixture was subjected to reaction at 37°C for 4 hours, followed by ethanol precipitation. The obtained precipitate was dissolved in 30 µl of a buffer [20 mM Tris-HCl (pH 8.5), 10 mM MgCl₂, 100 mM KCl, 1 mM DTT], and 10 units of BamHI was added thereto. The mixture was subjected to reaction at 37°C for 4 hours. The reaction mixture was subjected to agarose gel electrophoresis, and about 0.9 µg of DNA of ca. 9.6 kb was obtained.

The PstI-BamHI fragment derived from pHVC002 (50 ng) and the PstI-BamHI fragment derived from pVL1393 (200 ng) were dissolved in 20 µl of T4 DNA ligase buffer (the one attached to the enzyme was used), and 200 units T4 DNA ligase was added thereto. The mixture was subjected to ligation reaction at 12°C for 16 hours. The recombinant plasmid DNA obtained by the ligation was used to transform E. coli XL1-Blue, and plasmid pVL-HVC002 was obtained.

Then, 1 µg of pVL-HVC002 and 20 ng of filamentous baculovirus DNA (BaculoGold baculovirus DNA; Pharmingen) were dissolved in 12 µl of distilled water, followed by addition of a mixture of 6 µl of lipofectin and 6 µl of distilled water. The resulting mixture was allowed to stand at room temperature for 15 minutes. Separately, Sf9 cells (2 x 10⁶ cells) were suspended in 2 ml of Sf900-II medium (Life Technologies) and the suspension was put into a plastic Petri dish for culturing cells (diameter: 35 mm). To the Petri dish was added the whole of the above solution containing pVL-HVC002, the filamentous baculovirus DNA and lipofectin. After culturing at 27°C for 3 days, 1 ml of the culture supernatant containing the recombinant virus was separated. Then, 1 ml of fresh Sf900-II medium was added to the Petri dish, followed by culturing at 27°C for 3 days to obtain 1.5 ml of the supernatant containing the recombinant virus.

Sf9 cells (2 x 10⁷ cells) were suspended in 10 ml of Sf900-II medium and the suspension was allowed to stand at room temperature for one hour in a flask (75 cm², Gliner) to attach the cells thereto. After the supernatant was removed, 15 ml of TMN-FH medium for insect cells and 1 ml of the above supernatant containing the recombinant virus were added to the flask, followed by culturing at 27°C for 3 days. The culture supernatant was centrifuged at 1500 x g for 10 minutes to remove the cells, whereby a solution of the recombinant virus to be used for the expression of a protein was obtained.

The obtained recombinant virus solution was examined for virus titer in the following manner according to the instructions attached to BaculoGold Starter Kit. Sf9 cells (2 x 10⁶ cells) were suspended in 4 ml of Sf900-II medium and the suspension was allowed to stand at room temperature for one hour in a plastic Petri dish for culturing cells (diameter: 60 mm) to attach the cells thereto. After the supernatant was removed, 400 µl of Sf900-II medium and the above recombinant virus solution diluted 10000-fold with Sf900-II medium were added to the dish. The mixture was allowed to stand at room temperature for one hour, and then the medium was removed, followed by addition of 5 ml of a medium containing 1% low-gelling-temperature agarose (Agarplaque Agarose, Pharmingen) [a mixture of 1 ml of sterilized 5% aqueous solution of Agarplaque Agarose and 4 ml of TMN-FH medium for insect cells (Pharmingen) which had been kept at 42°C]. After the mixture was allowed to stand at room temperature for 15 minutes in the Petri dish, the dish was wound with a vinyl tape to avoid drying and then placed in a sealable plastic vessel. Culturing was carried out at 27°C for 6 days, and 1 ml of PBS containing 0.01% Neutral Red was added to the dish. After culturing for further one day, the number of plaques formed was counted. In this manner, the recombinant virus solution was found to contain the virus of about 2 x 10⁸ plaque-forming unit/ml (hereinafter referred to as PFU/ml).

### (2) Expression of the HVC002 Protein in Insect Cells

The HVC002 protein was expressed in the following manner according to the instructions attached to BaculoGold Starter Kit.

Sf21 cells (4 x 10⁷ cells) were put into a flask (175 cm²) to attach the cells thereto. After the culture supernatant was removed, 8 ml of Sf900-II medium and 2 ml of the solution containing the recombinant virus derived from pVL-HVC002 at a concentration of about 2 x 10⁸ PFU/ml obtained in (1) were added to the flask. Culturing was carried out at 27°C for 1-2 hours. After the culture supernatant was removed, 30 ml of Sf900-II medium was added, followed by culturing for 2-3 days. After the completion of culturing, the resulting culture was centrifuged at 1500 x g for 10 minutes to obtain the culture supernatant.

A column was charged with about 15 ml of Heparin-Sepharose fast flow gel (Pharmacia) and washed with 50 ml of buffer A [50 mM sodium phosphate (pH 6.5)] at a flow rate of 0.5 ml/min. Then, 500 ml of the culture supernatant containing the HVC002 protein prepared as above was passed through the column at a flow rate of 0.5 ml/min. After further washing with 80 ml of buffer A at a flow rate of 0.5 ml/min., elution was carried out using buffer A and buffer B [50 mM sodium phosphate (pH 6.5), 1 M NaCl] with NaCl concentration gradient of 0-1 M. The eluate was taken in 2 ml fractions and each fraction was subjected to Western blotting using monoclonal antibody KM1885 obtained in Example 5 (6) in the same manner as in Example 5 (4) to detect the HVC002 protein. As a result, 0.7-1.0 M NaCl fraction containing much HVC002 protein was obtained as a partially purified fraction.

The partially purified fraction was diluted with an equal amount of buffer A, and SP Sepharose (Pharmacia) was added thereto in an amount of 1% (v/v), followed by stirring at 4°C for 2 hours. A column was charged with the resin and washed with 10 times as much buffer C [50 mM sodium phosphate (pH 6.5), 0.4 M NaCl] as the resin by volume, followed by elution with 10 times as much buffer B as the resin by volume. The eluate was subjected to repeated concentrations using Centricon-3 (Amicon) and dilutions using buffer D [50 mM sodium phosphate (pH 7.3), 0.4 M NaCl] to obtain purified HVC002 protein. The purified HVC002 protein (0.5 µg) was subjected to 15% SDS-PAGE and silver staining, whereby a band which is considered to be a single band was obtained at the position of ca. 14.4 kDa as shown in Fig. 4.

### Example 7 Analysis of the N-terminal Amino Acid Sequence of the HVC002 Protein

The N-terminal amino acid sequence of the HVC002 protein was determined by an ordinary method used in protein chemistry. That is, the partially purified fraction obtained in Example 6 was subjected to SDS-PAGE with reduction with 2-mercaptoethanol and then electrically transferred onto a PVDF membrane (ProBlott; Perkin Elmer) according to the method of P. Matsudaira [J. Biol. Chem., 262, 10035 (1987)]. After the membrane was stained with Coomassie Blue, a band at about 8 kDa was cut out and the N-terminal amino acid sequence was analyzed by using a gas phase protein sequencer (PPSQ-10; Shimadzu Corporation) according to the method recommended by the manufacturer thereof. The obtained amino acid sequence was ThrXaaGlySerAspIleSerLys (SEQ ID NO: 11), which agreed with the sequence of amino acids 24 to 31 in the sequence of SEQ ID NO: 2 presumed from the nucleotide sequence. It was thus demonstrated that the open reading frame (ORF) having the sequence consisting of 94 amino acid residues shown by SEQ ID NO: 2 was translated from the mRNA and the signal sequence from the N-terminus to the 23rd amino acid residue was eliminated to result in the HVC002 protein having the sequence consisting of 71 amino acid residues shown by SEQ ID NO: 13 whose N-terminus is Thr which is the 24th amino acid residue in the ORF.

### Example 8 Analysis of Disulfide Bonds in the Molecule of the HVC002 Protein

The number of disulfide bonds in the molecule of the HVC002 protein is 0, 1 or 2, and the calculated values of molecular weight of the HVC002 protein are as follows according to the number of disulfide bonds: 0, 8397; 1, 8395; and 2, 8393. On the other hand, the purified HVC002 protein obtained in Example 6 was found to have the molecular weight of 8392±1 by the use of a mass spectrometer (LCQ; Thermoquest, USA). Accordingly, it was supposed that the molecule of the HVC002 protein contained two disulfide bonds, and their positions were determined in the following manner.

The purified HVC002 protein obtained in Example 6 was digested with trypsin (Sigma) and lysylendopeptidase (Wako Pure Chemical Industries, Ltd.) in 50 mM phosphate buffer (pH 7.3) containing 0.4 M sodium chloride. The reaction was carried out at 37°C for 9 hours with the substrate/enzyme ratio of 20 for trypsin and 50 for lysylendopeptidase. The reaction mixture was made acidic by addition of an appropriate amount of acetic acid and then subjected to reversed-phase HPLC under the following conditions.
Column: Vydac C8 (1 x 150 mm; Vydac);
Eluting solution A: 0.1% acetic acid, 0.02% trifluoroacetic acid;
Eluting solution B: 90% acetonitrile, 0.09% acetic acid, 0.02% trifluoroacetic acid;
Elution: linear concentration gradient from eluting solution A to eluting solution B (20 minutes);
Flow rate: 0.05 ml/min.

The elution of the peptides obtained by the enzyme digestion in reversed-phase HPLC was monitored by the absorbance at 214nm. The molecular weight of each peak having the absorbance at 214 nm was measured by using the above-mentioned mass spectrometer. On the basis of the amino acid sequence presumed from the nucleotide sequence, the molecular weights of the peptides formed by the cleavage at a lysine residue or an arginine residue as the carboxyl terminus were calculated and compared with the found values obtained by using the mass spectrometer. As a result, it was found that the peptide having the molecular weight of 4269±1 eluted in reversed-phase HPLC agreed with a peptide consisting of three constituent peptides shown in Table 1 bonded by intermolecular disulfide bonds.

**Table 1**

| Constituent peptide | Molecular weight (calcd.) |
|---|---|
| ValCysThrHisProArg | 711.8 |
| ThrCysCysPheGlnTyrSerHisLysProLeuProTrpThrTrpValArg | 2152.5 |
| SerTyrGluPheThrSerAsnSerCysSerGlnArg | 1408.5 |

There are two possible modes of disulfide bonds for the three constituent peptides in Table 1 as shown below by formulae 1 and 2, one of which is to be selected.

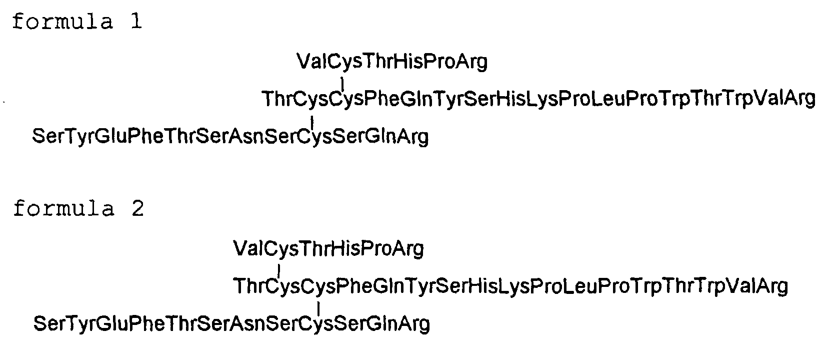

As reported by Morita and Nokihara [PSQ-BULLETIN, No. 12, p. 1-8 (1993), Shimadzu Corporation], disulfide bonds are stable in the Edman reaction, and cystine can be identified as phenylthiohydantoin (PTH)-cystine. N-terminal amino acid sequence analysis was made for each of formula 1 and formula 2, and the anticipated PTH-amino acids of cycles 1 to 3 are shown in Table 2. The N-terminal amino acid sequence of the peptide having the molecular weight of 4269±1 eluted in reversed-phase HPLC was analyzed by using the gas phase protein sequencer used in Example 7, and the result was obtained which agreed with that on formula 1. On the basis of this result, the intramolecular disulfide bonds of the HVC002 protein were identified to be Cys(10)-Cys(34) and Cys(11)-Cys(50) (the numbers in parentheses represent the positions in the amino acid sequence of SEQ ID NO: 2).

**Table 2**

| | Cycle 1 | Cycle 2 | Cycle 3 |
|---|---|---|---|
| Formula 1 | Val/Thr/Ser | Tyr | Thr/Cys-Cys/Glu |
| Formula 2 | Val/Thr/Ser | Tyr/Cys-Cys | Thr/Glu |
| Cys-Cys : represents cystine | | | |

In the HVC002 protein, disulfide bonds are formed between the first Cys and the third Cys, and between the second Cys and the fourth Cys. This mode of bonds is the same as that in MIP-1β [Science, 263, 1762 (1994)], RANTES [Biochemistry, 34, 9307 (1995)] and MCP-3 [Biochemistry, 36, 4412 (1997)], which are CC chemokines.

### Example 9 Calcium Mobilization Assay Using the Purified

### HVC002 Protein (1) Isolation of Human Eosinophils

To 30 ml of peripheral blood taken from a healthy human was added 40 ml of physiological saline (Otsuka Pharmaceutical Co., Ltd.) and the mixture was layered over 12 ml of Percoll (Pharmacia) the specific gravity of which had been adjusted to 1.085 g/ml. After centrifugation at 1500 rpm for 25 minutes at room temperature, the plasma layer, the mononuclear cell layer and the platelets were removed. To the residue was added 18 ml of sterile ice-cold water, followed by mixing for 30 seconds to cause hemolysis. Then, 2 ml of 10 x PIPES (64.3 g/l NaCl, 3.7 g/l KCl, 76 g/l PIPES, 0.42 N NaOH) was added, and the mixture was centrifuged at 1200 rpm at 4°C for 5 minutes. After the supernatant was discarded, 1 ml of 1 x PIPES was added and the hemolysis step was carried out again. After centrifugation, the supernatant was discarded and granulocytes isolated as the residual cells were washed with 10 ml of 2% FCS/PBS [PBS containing 2% FCS (fetal calf serum)]. The granulocytes were suspended in 2 ml of 2% FCS/PBS and anti-CD16 antibody (CLB) was added in an amount of 2 mg for 10⁷ cells of granulocytes, followed by stirring for 30 minutes under ice-cooling. Then, 10 ml of 2% FCS/PBS was added and the mixture was centrifuged. After the supernatant was discarded, the granulocytes were suspended in 1 ml of RPMI1640 medium containing 10% FCS. To the suspension were added anti-mouse IgG beads Dynabeades (Veritas) which had been washed twice with the same medium as above at the granulocytes:beads ratio of 1:4, and the mixture was shaken at 4°C for 45 minutes. The beads were drawn to a magnet and the supernatant was taken in fractions. To the beads was added 2 ml of RPMI1640 medium containing 10% FCS, followed by shaking. The supernatant was taken again, whereby about 5 x 10⁶ cells of eosinophils were obtained.

### (2) Culturing of HL60 (clone 15)

Culturing of myeloid cell line HL-60 (clone 15) (ATCC CRL-1964) and induction of differentiation thereof into eosinophil-like cells were carried out according to the report by Fischkoff, et al. [Cancer Research, 45, 2065 (1985)].

That is, HL-60 (clone 15) was cultured in RPMI1640 medium (pH 7.6) containing 10% FCS, 50 units/ml penicillin, 50 mg/ml streptomycin and 25 mM HEPES in a 5% CO₂ incubator at 37°C. The cultured cells were suspended in the same medium as above (pH 7.4) to a density of 5 x 10⁵ cells/ml, and butyric acid (Sigma) was added thereto at a concentration of 0.5 mM. By culturing in the incubator for 5 days, differentiation of the cells into eosinophil-like cells was induced.

### (3) Measurement of Calcium Mobilization Activity

The eosinophils prepared in (1) and the HL-60 (clone 15) cells differentiated into eosinophil-like cells in (2) were respectively washed with HACM buffer [20 mM Hepes-NaOH (pH 7.0), 125 mM NaCl, 5 mM KCl, 0.5 mM glucose, 0.025% BSA, 1 mM CaCl₂, 1 mM MgCl₂] and then suspended in the same buffer to a density of 1 x 10⁶ cells/ml. To 1 ml of the cell suspension was added 2 ml of a calcium indicator, Fra-2 mixture [a 5:2:2 mixture of 1 mM Fra-2/AM-DMSO solution (Wako Pure Chemical Industries, Ltd.), Cremophor EL (Sigma) and HACM buffer which had been subjected to ultrasonication for 20 seconds]. The resulting mixture was kept at 37°C for 30 minutes and centrifuged at 1000 rpm for 10 minutes. After the supernatant was discarded, the cells were washed with HACM buffer and then suspended in the same buffer to a density of 1 x 10⁶ cells/ml.

The intracellular calcium concentration was measured by using an intracellular ion measurement apparatus (CAF-110, Nippon Bunko Kogyo Co., Ltd.). The above cell suspension (500 µl) was put into a microcuvette and kept at 37°C for 3 minutes with stirring at 800 rpm. To the suspension were respectively added 5 µl of a solution of the purified HVC002 protein obtained in Example 6 (0.2 mg/ml) in a buffer [50 mM sodium phosphate (pH 7.3), 0.4 M NaCl] (final concentration of the HVC002 protein: ca. 0.2 µM) and 5 µl of a buffer [50 mM sodium phosphate (pH 7.3), 0.4 M NaCl]. The wavelengths for excitation were 340 nm and 380 nm and that for measurement was 500 nm.

As shown in Fig. 5, an increase in intracellular calcium concentration was observed when the HVC002 protein was added to the peripheral eosinophils and HL-60 differentiated into eosinophils, but was not observed by the addition of the control buffer alone. It was thus demonstrated that eosinophils react specifically with the HVC002 protein.

### Industrial Applicability

The present invention provides a protein capable of activating eosinophils; a DNA or an oligonucleotide coding for said protein; a recombinant vector comprising said DNA; a transformant carrying said recombinant vector; a process for producing a protein using said transformant; a cell which is specifically acted on by said protein; a cell membrane or a receptor which specifically binds to said protein; an agonist or an antagonist of said protein; an antibody which specifically binds to said protein; and a therapeutic agent or a diagnostic method for allergic inflammation, eosinophilic pneumonia, hypereosinophilic syndrome, autoimmune diseases, malignant tumors and parasitism utilizing them.

SEQUENCE LISTING

<110> KYOWA HAKKO KOGYO CO., LTD.
6-1, Ohtemachi 1-chome, Chiyoda-ku, Tokyo 100, Japan

<120> NOVEL DNA, NOVEL PROTEIN AND NOVEL ANTIBODY

<130> 11027

<140> PCT/JP97/04470
<141> 1997-12-05

<150> JP 96/325,762
<151> 1996-12-05

<160> 14

<170> PatentIn Ver. 2.0

SEQ ID NO: 1
<211> 282
<212> DNA
<213> Human

<400> 1
atg atg ggc ctc tcc ttg gcc tct gct gtg ctc ctg gcc tcc ctc ctg    48

Met Met Gly Leu Ser Leu Ala Ser Ala Val Leu Leu Ala Ser Leu Leu
1               5                   10                  15

agt ctc cac ctt gga act gcc aca cgt ggg agt gac ata tcc aag acc     96
Ser Leu His Leu Gly Thr Ala Thr Arg Gly Ser Asp Ile Ser Lys Thr
            20                  25                  30

tgc tgc ttc caa tac agc cac aag ccc ctt ccc tgg acc tgg gtg cga     144
Cys Cys Phe Gln Tyr Ser His Lys Pro Leu Pro Trp Thr Trp Val Arg
        35                  40                  45

agc tat gaa ttc acc agt aac agc tgc tcc cag cgg gct gtg ata ttc     192
Ser Tyr Glu Phe Thr Ser Asn Ser Cys Ser Gln Arg Ala Val Ile Phe
    50                  55                  60

act acc aaa aga ggc aag aaa gtc tgt acc cat cca agg aaa aaa tgg     240
Thr Thr Lys Arg Gly Lys Lys Val Cys Thr His Pro Arg Lys Lys Trp
65                  70                  75                  80

gtg caa aaa tac att tct tta ctg aaa act ccg aaa caa ttg             282
Val Gln Lys Tyr Ile Ser Leu Leu Lys Thr Pro Lys Gln Leu
                85                  90

SEQ ID NO: 2
<211> 94

<212> PRT

<213> Human

<400> 2

Met Met Gly Leu Ser Leu Ala Ser Ala Val Leu Leu Ala Ser Leu Leu

1               5                   10                  15

Ser Leu His Leu Gly Thr Ala Thr Arg Gly Ser Asp Ile Ser Lys Thr

            20                  25                  30

Cys Cys Phe Gln Tyr Ser His Lys Pro Leu Pro Trp Thr Trp Val Arg

        35                  40                  45

Ser Tyr Glu Phe Thr Ser Asn Ser Cys Ser Gln Arg Ala Val Ile Phe

    50                  55                  60

Thr Thr Lys Arg Gly Lys Lys Val Cys Thr His Pro Arg Lys Lys Trp

65                  70                  75                  80

Val Gln Lys Tyr Ile Ser Leu Leu Lys Thr Pro Lys Gln Leu

                85                  90

SEQ ID NO: 3

<211> 322

<212> DNA

<213> Human

<400> 3

tgtctgggtg cgaagctatg aattcaccag taacagctgc tcccagcggg ctgtgatatt 60

cactaccaaa agaggcaaga aagtctgtac ccatccaagg aaaaaatggg tgcaaaaata 120

catttcttta ctgaaaactc cgaaacaatt gtgactcagc tgaattttca tccgaggacg 180

cttggacccc gctcttggct ctgcagccct ctggggagcc tgcggaatct tttctgaagg 240

ctacatggac ccgctgggga ggagagggtg tttcctccca gagttacttt aataaaggtt 300

gttcataaaa aaaaaaaaaa ac 322

SEQ ID NO: 4
<211> 17
<212> DNA
<213> Artificial Sequence

<220>
<223> Description of Artificial Sequence: Synthetic DNA

<400> 4
gttttttttt ttttta 17

SEQ ID NO: 5
<211> 24
<212> DNA

<213> Artificial Sequence

<220>
<223> Description of Artificial Sequence: Synthetic DNA

<400> 5
cagcgggctg tgatattcac tacc                                                24

SEQ ID NO: 6
<211> 24
<212> DNA
<213> Artificial Sequence

<220>
<223> Description of Artificial Sequence: Synthetic DNA

<400> 6
tcagaaaaga ttccgcagct cccc                                                24

SEQ ID NO: 7
<211> 32
<212> DNA
<213> Artificial Sequence

<220>

<223> Description of Artificial Sequence: Synthetic DNA

<400> 7

tctctctcgc gacacgtggg agtgacatat cc                                    32

SEQ ID NO: 8

<211> 36

<212> DNA

<213> Artificial Sequence

<220>

<223> Description of Artificial Sequence: Synthetic DNA

<400> 8

tctctcggta cctcacaatt gtttcggagt tttcag                                36

SEQ ID NO: 9

<211> 22

<212> PRT

<213> Artificial Sequence

<220>

<223> Description of Artificial Sequence: Synthetic peptide

<400> 9

Cys Thr His Pro Arg Lys Lys Trp Val Gln Lys Tyr Ile Ser Leu Leu

1               5                   10                  15

Lys Thr Pro Lys Gln Leu

            20

SEQ ID NO: 10

<211> 11

<212> PRT

<213> Artificial Sequence

<220>

<223> Description of Artificial Sequence: Synthetic peptide

<400> 10

Ala Thr Arg Gly Ser Asp Ile Ser Lys Thr Cys

1               5                   10

SEQ ID NO: 11

<211> 8

<212> PRT

<213> Human

<400> 11

Thr Xaa Gly Ser Asp Ile Ser Lys

1               5

SEQ ID NO: 12

<211> 459

<212> DNA

<213> Human

<400> 12

ctgatttgca gcatc atg atg ggc ctc tcc ttg gcc tct gct gtg ctc ctg   51

                 Met Met Gly Leu Ser Leu Ala Ser Ala Val Leu Leu

                  1               5                   10

gcc tcc ctc ctg agt ctc cac ctt gga act gcc aca cgt ggg agt gac   99

Ala Ser Leu Leu Ser Leu His Leu Gly Thr Ala Thr Arg Gly Ser Asp

        15                  20                  25

ata tcc aag acc tgc tgc ttc caa tac agc cac aag ccc ctt ccc tgg   147

Ile Ser Lys Thr Cys Cys Phe Gln Tyr Ser His Lys Pro Leu Pro Trp

    30                  35                  40

acc tgg gtg cga agc tat gaa ttc acc agt aac agc tgc tcc cag cgg   195

Thr Trp Val Arg Ser Tyr Glu Phe Thr Ser Asn Ser Cys Ser Gln Arg

45                  50                  55                  60

gct gtg ata ttc act acc aaa aga ggc aag aaa gtc tgt acc cat cca 243
Ala Val Ile Phe Thr Thr Lys Arg Gly Lys Lys Val Cys Thr His Pro
                65                  70                  75

agg aaa aaa tgg gtg caa aaa tac att tct tta ctg aaa act ccg aaa 291
Arg Lys Lys Trp Val Gln Lys Tyr Ile Ser Leu Leu Lys Thr Pro Lys
            80                  85                  90

caa ttg tgactcagct gaattttcat ccgaggacgc ttggaccccg ctcttggctc 347
Gln Leu

tgcagccctc tggggagcct gcggaatctt ttctgaaggc tacatggacc cgctggggag 407

gagagggtgt ttcctcccag agttacttta ataaaggttg ttcatagagt tg 459

SEQ ID NO: 13
<211> 71
<212> PRT
<213> Human

<400> 13
Thr Arg Gly Ser Asp Ile Ser Lys Thr Cys Cys Phe Gln Tyr Ser His
1               5                   10                  15

Lys Pro Leu Pro Trp Thr Trp Val Arg Ser Tyr Glu Phe Thr Ser Asn
            20                  25                  30
Ser Cys Ser Gln Arg Ala Val Ile Phe Thr Thr Lys Arg Gly Lys Lys
        35                  40                  45
Val Cys Thr His Pro Arg Lys Lys Trp Val Gln Lys Tyr Ile Ser Leu
    50                  55                  60
Leu Lys Thr Pro Lys Gln Leu
65                  70

SEQ ID NO: 14
<211> 213
<212> DNA
<213> Human

<400> 14
aca cgt ggg agt gac ata tcc aag acc tgc tgc ttc caa tac agc cac 48
Thr Arg Gly Ser Asp Ile Ser Lys Thr Cys Cys Phe Gln Tyr Ser His
1               5                   10                  15

aag ccc ctt ccc tgg acc tgg gtg cga agc tat gaa ttc acc agt aac 96
Lys Pro Leu Pro Trp Thr Trp Val Arg Ser Tyr Glu Phe Thr Ser Asn
            20                  25                  30

agc tgc tcc cag cgg gct gtg ata ttc act acc aaa aga ggc aag aaa 144
Ser Cys Ser Gln Arg Ala Val Ile Phe Thr Thr Lys Arg Gly Lys Lys

        35                  40                  45

gtc tgt acc cat cca agg aaa aaa tgg gtg caa aaa tac att tct tta 192
Val Cys Thr His Pro Arg Lys Lys Trp Val Gln Lys Tyr Ile Ser Leu
    50                  55                  60

ctg aaa act ccg aaa caa ttg 213
Leu Lys Thr Pro Lys Gln Leu
65                  70

## Claims

1. A protein having the amino acid sequence represented by SEQ ID NO: 13, or a protein having an ability to activate eosinophils and having an amino acid sequence wherein one or more amino acid residues are deleted, substituted or added in the amino acid sequence represented by SEQ ID NO: 13.

2. The protein according to claim 1 having the amino acid sequence represented by SEQ ID NO: 2.

3. The protein according to claim 1 having an amino acid sequence which has 60% or more homology to the amino acid sequence represented by SEQ ID NO: 13.

4. The protein according to any of claims 1-3, said protein being a chemokine protein.

5. A DNA coding for the protein according to any of claims 1-4.

6. The DNA according to claim 5 having the nucleotide sequence represented by SEQ ID NO: 1, 12 or 14.

7. A DNA which hybridizes to the DNA according to claim 5 or 6 under stringent conditions and which codes for a protein having an ability to activate eosinophils.

8. A recombinant vector comprising the DNA according to any of claims 5-7.

9. A transformant which is obtained by introducing the recombinant vector according to claim 8 into a host cell.

10. A process for producing a protein which comprises culturing the transformant according to claim 9 in a medium, allowing the protein according to any of claims 1-4 to accumulate in the culture, and recovering said protein from the culture.

11. A pharmaceutical composition comprising the protein according to any of claims 1-4.

12. A therapeutic agent for malignant tumors or parasitism comprising the protein according to any of claims 1-4.

13. A method of treating malignant tumors or parasitism which comprises administering an effective amount of the protein according to any of claims 1-4.

14. A method for detecting a cell which is specifically acted on by the protein according to any of claims 1-4, which comprises bringing the protein according to any of claims 1-4 into contact with a test sample.

15. The method according to claim 14, wherein said test sample is a cell or a cell membrane.

16. The method according to claim 15, which comprises bringing the protein into contact with the cell and detecting the change in intracellular calcium concentration.

17. A method for detecting a cell which is specifically acted on by the protein according to claim 4, which comprises detecting the chemotaxis of leukocytes caused by the protein according to claim 4.

18. A method for obtaining a cell which is specifically acted on by the protein according to any of claims 1-4, which comprises bringing the protein according to any of claims 1-4 into contact with a test sample.

19. The method according to claim 18, wherein said test sample is a cell or a cell membrane.

20. The method according to claim 18, which comprises bringing the protein into contact with the cell and detecting the change in intracellular calcium concentration.

21. A method for obtaining a cell which is specifically acted on by the protein according to claim 4, which comprises detecting the chemotaxis of leukocytes caused by the protein according to claim 4.

22. A cell which is obtainable by the method according to any of claims 18-21.

23. A method for obtaining a cell membrane or a receptor which specifically binds to the protein according to any of claims 1-4, which comprises bringing the protein according to any of claims 1-4 into contact with a test sample.

24. The method according to claim 23, wherein said test sample is a cell, a cell membrane or a receptor.

25. The method according to claim 24, which comprises bringing the protein into contact with the cell, detecting the change in intracellular calcium concentration, and isolating the cell membrane or the receptor from the cell with which the change was detected.

26. A method for obtaining a cell membrane or a receptor which specifically binds to the protein according to claim 4, which comprises isolating the cell membrane or the receptor from a cell with which the chemotaxis of leukocytes caused by the protein according to claim 4 was detected.

27. The method according to any of claims 23-26, wherein said cell is an eosinophil.

28. A cell membrane or a receptor which is obtainable by the method according to any of claims 23-27.

29. A method for obtaining an agonist or an antagonist of the protein according to any of claims 1-4, which comprises comparing (a) the result obtained by bringing the protein according to any of claims 1-4 into contact with the cell according to claim 22 with (b) the result obtained by bringing the protein according to any of claims 1-4 into contact with the cell according to claim 22 and a test compound.

30. A method for obtaining an agonist or an antagonist of the protein according to any of claims 1-4, which comprises comparing (a) the result obtained by bringing the protein according to any of claims 1-4 into contact with the cell membrane or the receptor according to claim 28 with (b) the result obtained by bringing the protein according to any of claims 1-4 into contact with the cell membrane or the receptor according to claim 28 and a test compound.

31. An agonist or an antagonist which is obtainable by the method according to claim 29 or 30.

32. An antibody which specifically reacts with the protein according to any of claims 1-4.

33. A polyclonal antibody which specifically reacts with the protein according to any of claims 1-4.

34. A monoclonal antibody which specifically reacts with the protein according to any of claims 1-4.

35. The monoclonal antibody according to claim 34 which recognizes the amino acid sequence represented by SEQ ID NO: 9 as the epitope.

36. The monoclonal antibody according to claim 35, said monoclonal antibody being monoclonal antibody KM1885 which is produced by hybridoma KM1885 (FERM BP-6177).

37. Hybridoma KM1885 which produces the monoclonal antibody according to claim 36.

38. A method for immunologically detecting the protein according to any of claims 1-4 which is characterized by the use of the antibody according to any of claims 32-36.

39. A diagnostic method for allergic inflammation, eosinophilic pneumonia, hypereosinophilic syndrome or autoimmune diseases which is characterized by the use of the antibody according to any of claims 32-36.

40. A diagnostic agent for allergic inflammation, eosinophilic pneumonia, hypereosinophilic syndrome or autoimmune diseases comprising the antibody according to any of claims 32-36.

41. A pharmaceutical composition comprising the antibody according to any of claims 32-36.

42. A therapeutic agent for allergic inflammation, eosinophilic pneumonia, hypereosinophilic syndrome or autoimmune diseases comprising the antibody according to any of claims 32-36.

43. A method of treating allergic inflammation, eosinophilic pneumonia, hypereosinophilic syndrome or autoimmune diseases which comprises administering an effective amount of the antibody according to any of claims 32-36.

44. The DNA according to claim 5 or 6, said DNA being a sense DNA.

45. The DNA according to claim 5 or 6, said DNA being an antisense DNA.

46. An oligonucleotide comprising a part of the nucleotide sequence of the DNA according to claim 44.

47. An oligonucleotide comprising a part of the nucleotide sequence of the DNA according to claim 45.

48. A method for detecting an mRNA coding for the protein according to any of claims 1-4 which is characterized by the use of the DNA according to claim 44 or 45 or the oligonucleotide according to claim 46 or 47.

49. A diagnostic method for allergic inflammation, eosinophilic pneumonia, hypereosinophilic syndrome, autoimmune diseases, malignant tumors or parasitism which is characterized by the use of the DNA according to claim 44 or 45 or the oligonucleotide according to claim 46 or 47.

50. A diagnostic agent for allergic inflammation, eosinophilic pneumonia, hypereosinophilic syndrome, autoimmune diseases, malignant tumors or parasitism comprising the DNA according to claim 44 or 45 or the oligonucleotide according to claim 46 or 47.

51. A method for repressing the expression of the protein according to any of claims 1-4 which is characterized by the use of the DNA according to claim 45 or the oligonucleotide according to claim 47.

52. A pharmaceutical composition comprising the DNA according to claim 45 or the oligonucleotide according to claim 47.

53. A therapeutic agent for allergic inflammation, eosinophilic pneumonia, hypereosinophilic syndrome or autoimmune diseases comprising the DNA according to claim 45 or the oligonucleotide according to claim 47.

54. A method of treating allergic inflammation, eosinophilic pneumonia, hypereosinophilic syndrome or autoimmune diseases which comprises administering an effective amount of the DNA according to claim 45 or the oligonucleotide according to claim 47.

55. A vector for the gene therapy for allergic inflammation, eosinophilic pneumonia, hypereosinophilic syndrome, autoimmune diseases, malignant tumors or parasitism comprising the DNA according to claim 44 or 45 or the oligonucleotide according to claim 46 or 47.

56. A method of treating allergic inflammation, eosinophilic pneumonia, hypereosinophilic syndrome, autoimmune diseases, malignant tumors or parasitism which is characterized by the use of the vector for the gene therapy according to claim 55.

57. A method for obtaining a DNA coding for a chemokine protein from human vascular endothelial cells stimulated with IL-4 by using the differential display technique.

58. The method according to claim 57, wherein the chemokine protein is the protein according to claim 4.
